# EUROPEAN PATENT APPLICATION

(11) **EP 4 785 906 A1**
(43) Date of publication of application: **05.08.2026**
(21) Application number: 23958098.8
(22) Date of filing: 23.11.2023
(51) Int. Cl.: A61F 2/24

(54) **VALVE CLAMPING DEVICE AND VALVE REPAIR SYSTEM**

(30) Priority: 06.11.2023 CN 202311468173
(71) Applicant: Shanghai Push Medical Device Co., Ltd., Shanghai 200331 (CN)
(72) Inventor: LI, Rui, Shanghai 200331 (CN); GONG, Shanshi, Shanghai 200331 (CN); YAN, Wei, Shanghai 200331 (CN); JI, Jiadong, Shanghai 200331 (CN)
(74) Representative: Canzler & Bergmeier Patentanwälte Partnerschaft mbB
(86) International application number: PCT/CN2023/133712
(87) International publication number: WO 2025/097497

(57) **Abstract**

Disclosed are a valve clamping device (100) and a valve repair system. The valve clamping device (100) comprises a main body (110) and a clamping body (120). The clamping body (120) comprises a clamping member (121), and is mounted on the main body (110). The main body (110) comprises a transmission assembly (111). The transmission assembly (111) comprises a first connecting rod (1111) and a second connecting rod (1112). A distal end of the first connecting rod (1111) and a proximal end of the second connecting rod (1112) are provided with a transmission structure (1113). The transmission structure (1113) transmits axial movement between the first connecting rod (1111) and the second connecting rod (1112). The clamping member (121) is hinged to a distal end of the second connecting rod (1112), and axial movement of the second connecting rod (1112) drives opening and closing movement of the clamping member (121), changing an included angle between the clamping member (121) and the main body (110). The described segmented transmission structure isolates transmission of radial movement tendency from a first segment of the main body (110) to a second segment, thereby enabling the second segment to drive the opening and closing movement of the clamping member (121), causing movement of the clamping member (121) to tend toward continuous and stable changes. During operation, the valve clamping device (100) maintains a more stable state, which is beneficial for the operator to control the clamping member (121), and reduces operational difficulty.

## Description

### Background of the Present Disclosure

### FIELD OF THE DISCLOSURE

The present disclosure relates to the technical field of medical devices, and in particular to a valve clamping device and a valve repair system.

### DESCRIPTION OF RELATED ARTS

Medical devices play an increasingly important role in the diagnosis, treatment, and other links of diseases. Transcatheter interventional therapy can achieve a better therapeutic effect with a smaller wound and is more conducive to postoperative recovery, thus having achieved rapid development. When an operator delivers an implanted medical device through a catheter and operates the implanted medical device, the operation is often guided by images. However, challenges of the complex environment in the patient's body still exist. It is necessary to continuously improve the medical devices and enhance their performance.

### SUMMARY OF THE PRESENT DISCLOSURE

The present disclosure provides a valve clamping device and a valve repair system with better performance, so as to improve the effect of transcatheter interventional therapy.

In a first aspect, the present disclosure provides a valve clamping device, including a main body and a clamping body, wherein the clamping body is mounted on the main body; the main body includes a transmission assembly, the transmission assembly includes a first transmission rod and a second transmission rod, a transmission structure is arranged at the distal end of the first transmission rod and the proximal end of the second transmission rod, and the transmission structure transmits axial motion between the first transmission rod and the second transmission rod; the clamping body includes a clamping member, the clamping member is hinged to the distal end of the second transmission rod, and the axial motion of the second transmission rod drives the opening and closing motions of the clamping member, so as to change the included angle between the clamping member and the main body.

In one embodiment, the driving force for controlling the valve clamping device acts radially on the first transmission rod, and the transmission structure transmits the axial motion while preventing the transmission of radial motion tendency to the second transmission rod.

In one embodiment, the transmission structure includes a first limiting structure and a second limiting structure; the first limiting structure is arranged at the distal end of the first transmission rod, the second limiting structure is arranged at the proximal end of the second transmission rod, and the first limiting structure and the second limiting structure axially abut against each other when the first transmission rod or the second transmission rod moves axially.

In one embodiment, the axial abutment between the first limiting structure and the second limiting structure restricts the relative movement between the first transmission rod and the second transmission rod in one axial direction, or the axial abutment between the first limiting structure and the second limiting structure restricts the relative movement between the first transmission rod and the second transmission rod in two axial directions.

In one embodiment, the second limiting structure includes a concave side surface, the first limiting structure includes an extension part extending towards the interior of the first transmission rod, and the extension part is configured to be located in the groove of the concave side surface.

In one embodiment, the first limiting structure includes a first limiting member arranged at the distal end of the first transmission rod, the first limiting member includes a first chamber, the first chamber includes a first opening towards the distal end of the valve clamping device, the proximal end of the second transmission rod is configured to pass through the first opening towards the distal end of the valve clamping device, the second limiting structure at the proximal end of the second transmission rod is configured to be accommodated in the first chamber, and the maximum size of the second limiting structure is larger than the size of the first opening towards the distal end of the valve clamping device.

In one embodiment, the second limiting structure includes a second limiting member arranged at the proximal end of the second transmission rod, the second limiting member has a second chamber, the second chamber includes a second opening towards the proximal end of the valve clamping device, the distal end of the first transmission rod is configured to pass through the opening towards the proximal end of the valve clamping device, the first limiting structure at the distal end of the first transmission rod is configured to be accommodated in the second chamber, and the maximum size of the first limiting structure is larger than the size of the second opening towards the proximal end of the valve clamping device.

In one embodiment, the second limiting structure which is configured to be located in the first chamber of the first limiting member is spherical or ellipsoidal; or, the first limiting structure which is configured to be located in the second chamber of the second limiting member is spherical or ellipsoidal.

In one embodiment, the first limiting structure includes a third limiting member arranged at the distal end of the first transmission rod, the third chamber of the third limiting member further includes a third opening towards the proximal end of the valve clamping device, and the distal end of the first transmission rod is configured to pass through the third opening towards the proximal end of the valve clamping device and abut against the second limiting structure at the proximal end of the second transmission rod in the third chamber during axial motion towards the distal end of the valve clamping device.

In one embodiment, the second limiting structure includes a fourth limiting member arranged at the proximal end of the second transmission rod, the fourth chamber of the fourth limiting member further includes a fourth opening towards the distal end of the valve clamping device, and the proximal end of the second transmission rod is configured to pass through the fourth opening towards the distal end of the valve clamping device and abut against the first limiting structure at the proximal end of the first transmission rod in the fourth chamber during axial motion towards the distal end of the valve clamping device.

In one embodiment, the first limiting structure is integrally formed with the first transmission rod, and the second limiting structure is integrally formed with the second transmission rod; or, the first limiting structure is integrally formed with the first transmission rod, and the second limiting structure is fixedly connected to the second transmission rod; or, the first limiting structure is fixedly connected to the first transmission rod, and the second limiting structure is integrally formed with the second transmission rod; or, the first limiting structure is fixedly connected to the first transmission rod, and the second limiting structure is fixedly connected to the second transmission rod.

In one embodiment, at least one of the surfaces in the transmission assembly abutting against each other is provided with a convex structure.

In one embodiment, the first transmission rod includes an inner chamber, and the cross-section of the inner chamber is non-circular.

In one embodiment, the cross-section of the inner chamber is elliptical, rectangular, triangular, or racetrack-shaped.

In one embodiment, the main body further includes an attachment member, the first transmission rod is arranged in the attachment member, the inner side wall of the attachment member is provided with an internal thread, the outer side wall of the first transmission rod is provided with an external thread, and the internal thread matches the helix of the external thread.

In one embodiment, the clamping body further includes a capturing member arranged between the main body and the clamping member.

In a second aspect, the present disclosure provides a valve repair system, including the valve clamping device according to any one of the above embodiments in the first aspect, and a delivery system for delivering and controlling the valve clamping device.

In one embodiment, the delivery system includes a catheter and a delivery device, the delivery device includes a control mechanism and a delivery rod, the proximal end of the delivery rod is connected to the control mechanism, the distal end of the delivery rod passes through the catheter and acts radially on the transmission assembly of the valve clamping device, and the cross-section of the distal end of the delivery rod is non-circular.

In one embodiment, the surface of the delivery rod abutting against the second transmission rod of the transmission assembly of the valve clamping device is provided with a convex structure.

The above valve clamping device and valve repair system adopt a segmented transmission configuration, which isolates the transmission of radial motion tendency from the first section to the second section of the main body. In this way, the second section drives the opening and closing motions of the clamping member, so that the motion of the clamping member tends to change continuously and stably. During operation, the valve clamping device has a more stable state, which facilitates the operator's control over the clamping member and reduces the operation difficulty.

### BRIEF DESCRIPTION OF THE DRAWINGS

The drawings used in the embodiments of the present disclosure are briefly introduced below:
Figure 1 is a schematic diagram of a therapeutic application scenario to which an embodiment of the present disclosure is applied;
Figure 2 is a schematic structural diagram of a valve repair system according to an embodiment of the present disclosure;
Figure 3 is a schematic structural diagram of an enlarged valve clamping device at the distal end of the valve repair system according to an embodiment of the present disclosure;
Figure 4 is a plan view of the valve clamping device in a closed state according to an embodiment of the present disclosure;
Figure 5 is a plan view of the valve clamping device in an open state according to an embodiment of the present disclosure;
Figure 6 is a plan view of the valve clamping device in another open state according to an embodiment of the present disclosure;
Figure 7 is a plan view of the valve clamping device in an inverted state according to an embodiment of the present disclosure;
Figure 8 to Figure 14 are schematic diagrams of the valve clamping device during operation according to an embodiment of the present disclosure;
Figure 15 is a cross-sectional view of an embodiment of the main body of the valve clamping device shown in Figure 4 along the line A-A;
Figure 16 is a cross-sectional view of an embodiment of the main body of the valve clamping device shown in Figure 5 or Figure 6 along the line A-A;
Figure 17 is a cross-sectional view of an embodiment of the main body of the valve clamping device shown in Figure 7 along the line A-A;
Figure 18 is a plan view of a transmission assembly according to an embodiment of the present disclosure;
Figure 19 is a plan view of another transmission assembly according to an embodiment of the present disclosure;
Figure 20 is a plan view of yet another transmission assembly according to an embodiment of the present disclosure;
Figure 21 is a plan view of yet another transmission assembly according to an embodiment of the present disclosure;
Figure 22 is a plan view of yet another transmission assembly according to an embodiment of the present disclosure;
Figure 23 is a plan view of a limiting structure according to an embodiment of the present disclosure;
Figure 24 is a plan view of another limiting structure according to an embodiment of the present disclosure;
Figure 25 is a cross-sectional view of the valve clamping device when the delivery rod is inserted according to an embodiment of the present disclosure;
Figure 26 is a cross-sectional view of another valve clamping device according to an embodiment of the present disclosure;
Figure 27 is a cross-sectional view of yet another valve clamping device according to an embodiment of the present disclosure;
Figure 28 is a plan view of a convex structure according to an embodiment of the present disclosure;
Figure 29 is a plan view of another convex structure according to an embodiment of the present disclosure;
Figure 30 is a plan view of yet another convex structure according to an embodiment of the present disclosure;
Figure 31 is a schematic perspective view of the valve clamping device according to an embodiment of the present disclosure;
Figure 32 is a schematic perspective view of a clamping member according to an embodiment of the present disclosure;
Figure 33 is a plan view of the clamping member shown in Figure 32;
Figure 34 is a schematic perspective view of another clamping member according to an embodiment of the present disclosure;
Figure 35 is a side view of yet another clamping member according to an embodiment of the present disclosure;
Figure 36 is another side view of the clamping member shown in Figure 35;
Figure 37 is a schematic perspective view of yet another valve clamping device connected to a clutch device according to an embodiment of the present disclosure;
Figure 38 is a schematic perspective view of a support member according to an embodiment of the present disclosure;
Figure 39 is a schematic perspective view of another support member according to an embodiment of the present disclosure;
Figure 40 is a cross-sectional view of the support member according to an embodiment of the present disclosure;
Figure 41 is a plan view of yet another support member according to an embodiment of the present disclosure;
Figure 42 is a schematic perspective view of a capturing member according to an embodiment of the present disclosure;
Figure 43 is a side view of the capturing member shown in Figure 42;
Figure 44 is a schematic perspective view of another capturing member according to an embodiment of the present disclosure;
Figure 45 is a side view of the capturing member shown in Figure 44;
Figure 46 is a schematic perspective view of yet another capturing member according to an embodiment of the present disclosure;
Figure 47 is a schematic perspective view of another valve clamping device according to an embodiment of the present disclosure;
Figure 48 is a schematic perspective view of the support member with the capturing member mounted thereon shown in Figure 47 from another perspective;
Figure 49 is a schematic perspective view of yet another support member according to an embodiment of the present disclosure;
Figure 50 is a side view of another support member with a capturing member mounted thereon according to an embodiment of the present disclosure;
Figure 51 is a schematic perspective view of the clutch device in a natural state according to an embodiment of the present disclosure;
Figure 52 is a schematic perspective view of the clutch device under the action of external force according to an embodiment of the present disclosure;
Figure 53 is a side view of the clutch device according to an embodiment of the present disclosure;
Figure 54 is a schematic perspective view of a connecting structure on the main body according to an embodiment of the present disclosure;
Figure 55 is a schematic perspective view of another connecting structure on the main body according to an embodiment of the present disclosure;
Figure 56 is a schematic perspective view of the clutch device connected to the valve clamping device according to an embodiment of the present disclosure;
Figure 57 is a side view of the clutch device connected to the valve clamping device shown in Figure 56; and
Figure 58 is a schematic perspective view of another clutch device connected to the valve clamping device according to an embodiment of the present disclosure.

### DETAILED DESCRIPTION OF THE PREFERRED EMBODIMENTS

In order to more clearly illustrate the embodiments of the present disclosure or the technical solutions in the prior art, the specific implementations of the present disclosure will be described below with reference to the accompanying drawings. The drawings described below are only some embodiments of the present disclosure. For those of ordinary skill in the art, other drawings can be obtained according to these drawings without creative efforts, and other implementations obtained thereby, as well as adjustments and improvements made without departing from the concept of the present disclosure, all fall within the protection scope of the present disclosure.

For the sake of brevity of the drawings, each drawing in the embodiments of the present disclosure only schematically shows the parts related to the present disclosure, and they do not represent the actual structure of the product. In addition, to make the drawings concise and easy to understand, in some drawings, components with the same structure or function are only schematically shown in part, and more or fewer components with the same structure or function may actually exist.

In the present disclosure, unless otherwise expressly specified and limited, ordinal numbers such as "first" and "second" are only used to distinguish the described associated objects, and cannot be understood as indicating or implying the relative importance or sequence between the associated objects; in addition, they do not represent the quantity of the associated objects. "And/or" is used to describe the relationship between associated objects, including any relationship of the associated objects. For example, "a and/or b" includes: "a alone", "b alone", and "a and b". The terms "mounted" and "connected" should be interpreted in a broad sense. For example, "mounted" may be direct mounting or mounting via other components; "connected" may be direct connection or connection via other components. The term "arranged oppositely" includes parallel opposition or opposition at a certain angle, and the angle is not limited, which is determined according to the number of objects arranged oppositely.

In the embodiments shown in the drawings, the indication of directions (such as up, down, left, right, front, back, etc.) is not absolute but relative when describing the structure and movement of each component, and is not used to limit the actual use direction of the product.

In the embodiments of the present disclosure, the "proximal end" refers to the end of the associated object close to the operator; the "distal end" refers to the end of the associated object away from the operator. "Proximal end" and "distal end" are from the perspective of the operator (such as a doctor) using the equipment where the associated object is located (such as a medical device), and refer to the position or direction of the associated object (such as a component of the medical device) relative to the operator. For example, the "proximal end" refers to the end close to the doctor during the normal operation of the medical device by the doctor, and the "distal end" refers to the end away from the doctor during the normal operation of the medical device by the doctor, that is, the end that first enters the patient's body.

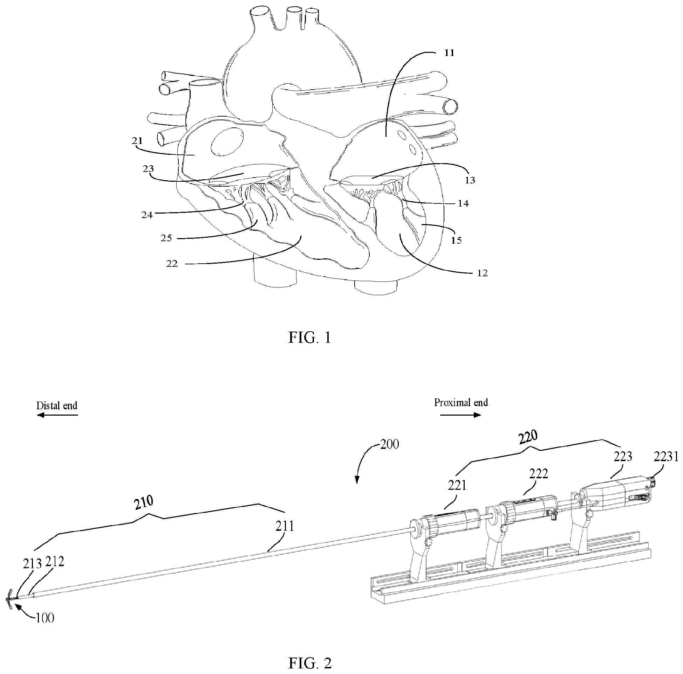

Medical devices play an increasingly important role in the diagnosis, treatment and other links of diseases. Transcatheter interventional therapy can achieve a therapeutic effect with a smaller wound and is more conducive to postoperative recovery, thus having achieved rapid development. For example, please refer to Figure 1, which is a schematic diagram of a therapeutic application scenario to which the embodiment of the present disclosure is applied, showing the structure of the heart: the heart is a hollow muscular organ with four chambers, namely the left atrium (LA) 11, right atrium (RA) 21, left ventricle (LV) 12, and right ventricle (RV) 22. An atrioventricular valve (hereinafter referred to as the valve) is located between the atrium and the ventricle. When the ventricle relaxes, the valve opens, and blood flows from the atrium into the ventricle; when the ventricle contracts, the valve closes to prevent blood from flowing back from the ventricle to the atrium. The valve between the left atrium 11 and the left ventricle 12 is the mitral valve (MV) 13, and the valve between the right atrium 21 and the right ventricle 22 is the tricuspid valve (TV) 23. The mitral valve 12 has two leaflets hanging vertically into the ventricular cavity and connected to the papillary muscles 15 on the left ventricular wall through chordae tendineae 14. The tricuspid valve 23 has three leaflets hanging vertically into the ventricular cavity and connected to the papillary muscles 25 on the right ventricular wall through chordae tendineae 24. When the valve cannot open and close normally, it will affect the circulating flow of blood. For example, mitral regurgitation or tricuspid regurgitation is an extremely common type of valvular lesion. In this type of lesion, an opening that cannot be closed appears at the center or one side of the coaptation edge when the valve is closed. Edge-to-edge repair is a treatment method for the above lesions, that is, the valve leaflets are opposed at the opening to reduce the opening area of the valve when closed and reduce the blood regurgitation volume when the valve is closed.

When edge-to-edge repair is performed for mitral regurgitation or tricuspid regurgitation using transcatheter interventional therapy, the operator performs precise repair of the valve through the catheter under the guidance of images (such as ultrasound). Due to the complex environment at the valve, the normal beating of the heart during the operation and other factors, the performance improvement of the valve clamping device for clamping the valve is particularly important.

The following description is made with reference to the accompanying drawings:

Please refer to Figure 2, which is a schematic structural diagram of a valve repair system according to an embodiment of the present disclosure. As shown in Figure 2, the valve repair system includes a valve clamping device 100 and a delivery system 200, and the delivery system 200 is used for delivering and controlling the valve clamping device 100. The delivery system 200 includes a catheter 210 and a delivery device 220. The distal end of the catheter 210 is detachably connected to the valve clamping device 100 under the control of the delivery device 220. When the delivery device 220 delivers the valve clamping device 100 to a required position through the catheter 210, after controlling the valve clamping device 100 to complete the capture and coaptation clamping of the valve leaflets, it controls the valve clamping device 100 to disengage from the distal end of the catheter 210 and remain in the patient's body.

In one embodiment, the delivery system 200 may adopt a three-layer structure. The catheter 210 (also referred to as a delivery sheath) includes an outer guide catheter 211 (also referred to as a guide sheath), a middle manipulation catheter 212 (also referred to as a manipulation sheath), and an inner implantation catheter 213 (also referred to as an implantation sheath). The proximal end of the catheter 210 can control the movement of the distal end of the catheter 210 through a control handle. For example, as shown in Figure 2, the delivery system 200 includes a guide catheter handle 221, a manipulation catheter handle 222, and an implantation catheter handle 223, which are respectively arranged at the proximal ends of the guide catheter 211, the manipulation catheter 212 and the implantation catheter 213, and are used for controlling the movement of the distal ends of the guide catheter 211, the manipulation catheter 212 and the implantation catheter 213.

During operation, the guide catheter handle 221 controls the movement of the distal end of the guide catheter 211, so that the guide catheter 211 enters the patient's body and provides an operation channel for the manipulation catheter 212; the manipulation catheter handle 222 controls the movement of the distal end of the manipulation catheter 212 to provide an operation channel for the implantation catheter 213. The guide catheter 211 and the manipulation catheter 212 are, for example, steerable catheters, which can achieve omnidirectional bending adjustment in the anterior-posterior and medial-lateral directions, bringing flexibility and convenience to the operation. The valve clamping device 100 is detachably arranged at the distal end of the implantation catheter 213, and the implantation catheter handle 223 controls the movement of the distal end of the implantation catheter 213 to deliver the valve clamping device 100 to the lesion. A delivery rod (not shown in Figure 2, see 214 in subsequent drawings) can be arranged in the implantation catheter 213, and a control mechanism 2231 (such as a control handle) can be arranged on the implantation catheter handle 223. This control mechanism 2231 is connected to the proximal end of the delivery rod, and the distal end of the delivery rod provides driving force to the valve clamping device 100 to control the state of the valve clamping device 100, so as to realize the capture and coaptation clamping of the valve leaflets by the valve clamping device 100. Further, other control mechanisms can be arranged on the implantation catheter handle 223 for controlling other operations of the valve clamping device 100. For example, a control mechanism for controlling the state of the capturing member of the valve clamping device 100 may be also provided to control the capturing member to open towards the clamping member during the capture of the valve leaflets so as to capture the valve leaflets; for another example, a control mechanism for controlling the connection state between the valve clamping device 100 and the distal end of the implantation catheter 213 may be also provided, which is used for controlling the valve clamping device 100 to disengage from the distal end of the implantation catheter 213 after completing the capture and coaptation clamping of the valve leaflets.

Further, the above delivery system 200 can be arranged on a bracket, which can provide support for the delivery system 200 and improve the stability of the valve repair system.

The implementation of the above delivery system 200 is only an example. In other embodiments, other structures can be adopted. For example, the control handle and the control mechanism on the control handle can be adjusted, so that the functions of part of the control handles are integrated, or the control mechanism on the control handle is further independently arranged, or part of the control functions are integrated or separated on the control handle, etc. The present disclosure is not limited to the above structure of the delivery system 200.

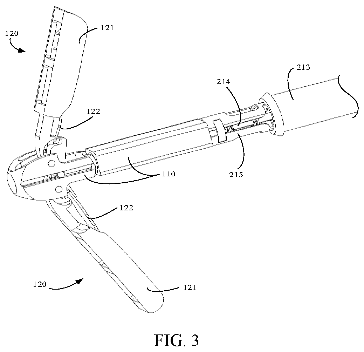

The valve clamping device is described below with reference to the accompanying drawings. Please refer to Figure 3, which is a schematic structural diagram of an enlarged valve clamping device at distal end of the valve repair system according to the embodiment of the present disclosure. To better reflect the structure of the valve clamping device, the covering film is not shown thereon. As shown in Figure 3, the valve clamping device 100 includes a main body 110 and a clamping body 120, wherein the main body 110 is used for mounting the clamping body 120, that is, the clamping body 120 is mounted on the main body 110; in other words, the main body 110 is used for providing support for the clamping body 120. Further, the clamping body 120 can perform opening and closing motions relative to the main body 110. The clamping body 120 includes clamping members 121 (also referred to as a clamping arm) and capturing members 122 (also referred to as a capturing arm). Both the clamping member 121 and the capturing member 122 can perform opening and closing motions relative to the main body 110, and their opening and closing motions can be independent of each other. Thus, when the clamping member 121 is opened relative to the main body 110, the capturing member 122 can be closed, and a space can be formed between the clamping member 121 and the capturing member 122 to facilitate the capture of the valve leaflets. When the valve leaflets are located between the clamping member 121 and the capturing member 122, the capturing member 122 can be controlled to open towards the clamping member 121, so as to clamp the valve leaflets between the capturing member 122 and the clamping member 121 to realize the capture of the valve leaflets; then, the clamping body 120 is further controlled to be clamped with the main body 110 (i.e., the clamping body 120 is controlled to perform a closing motion) to complete the coaptation clamping of the valve leaflets. The proximal end of the main body 110 is detachably connected to the distal end of the delivery system 200 through a clutch device 215 (for example, detachably connected to the distal end of the implantation catheter 213 of the delivery system 200). When the clamping body 110 completes the coaptation clamping of the valve leaflets, the main body 110 can be controlled to disengage from the distal end of the delivery system 200, so that the valve clamping device 100 remains in the patient's body to complete the repair of the valve.

The number of clamping body may be two or more. The present disclosure does not limit the number of clamping bodies. For the convenience of description, two are taken as an example here. In other embodiments, there can be more clamping bodies, and the implementation of other clamping bodies is the same as that described in the embodiment of the present disclosure, which will not be repeated here.

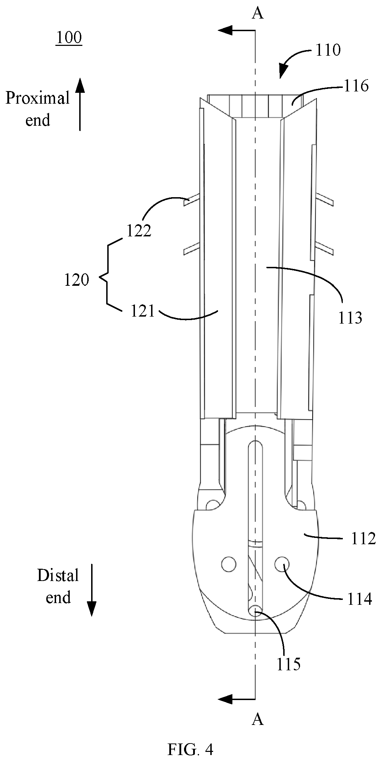

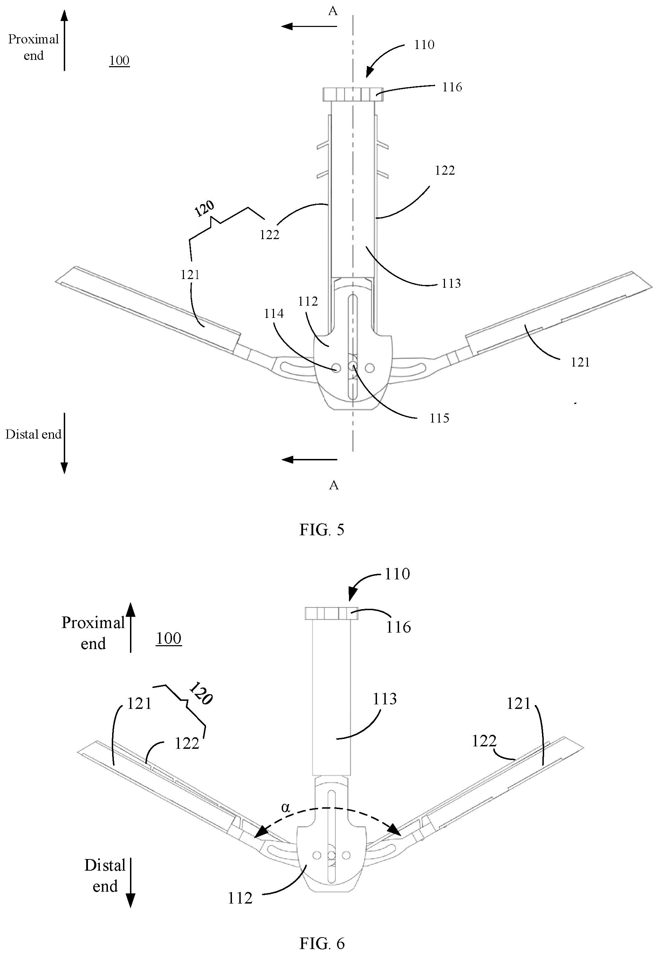

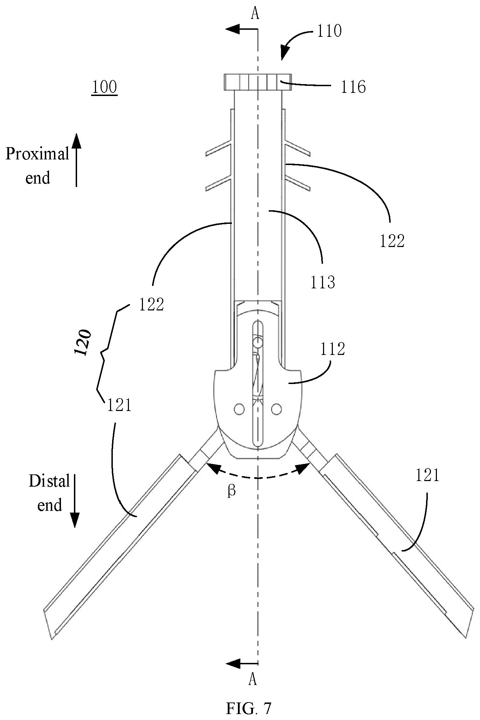

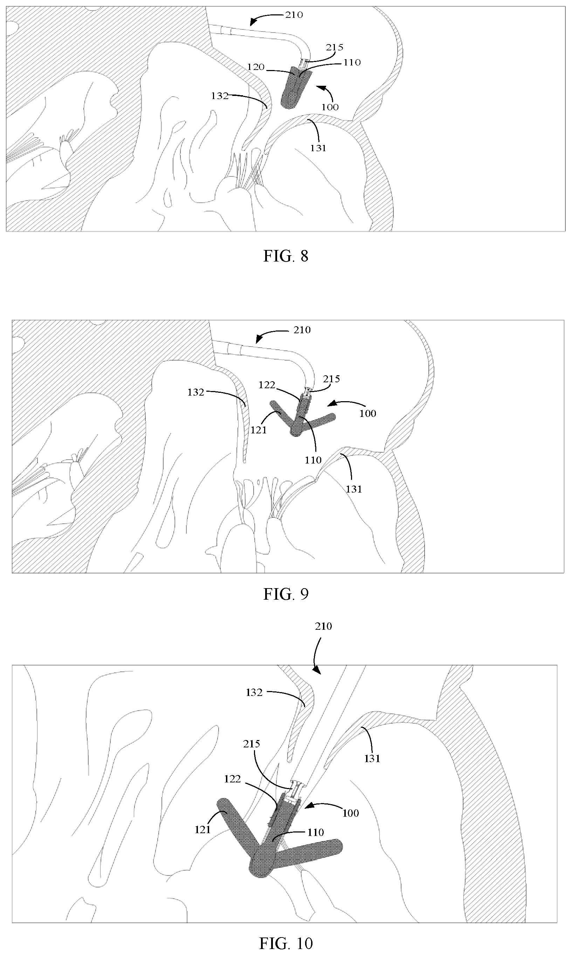

The valve clamping device 100 has a closed state and an open state, wherein the open state includes an inverted state. Taking mitral valve repair as an example, the different states of the valve clamping device 100 during the repair process are further described below in conjunction with Figure 4 to Figure 14. The valve clamping device 100 in Figure 4 to Figure 7 is not covered with a film to better show its structure; the valve clamping device 100 in Figure 8 to Figure 14 is covered with a film to better show its state during the repair process. The repair of other valve structures (such as the tricuspid valve) is similar thereto. Figure 4 is a plan view of the valve clamping device in a closed state; Figure 5 to Figure 7 are plan views of the valve clamping device in different open states, respectively, wherein the open state shown in Figure 7 can also be referred to as an inverted state. Figure 8 to Figure 14 are schematic diagrams of the valve clamping device during operation.

During the delivery of the valve clamping device 100, the valve clamping device 100 is in a closed state, as shown in Figure 4 and Figure 8. The delivery system delivers the valve clamping device 100 to the lesion through the catheter 210, that is, the position close to the leaflets 131 and 132 of the mitral valve. In the closed state, the clamping body 120 is clamped to (or attached to) the main body 110, that is, the clamping member 121 and the capturing member 122 on the same side are butted together and both are close to the main body 110. At this time, the valve clamping device 100 can enter the patient's body with the smallest radial size, which can effectively reduce the damage of the valve clamping device 100 to the patient and relieve the discomfort of the patient.

As shown in Figure 9, when the valve clamping device 100 is delivered to the target position (for example, the lesion), the clamping member 121 is controlled to open gradually, that is, the included angle with the main body 110 increases gradually, and the process of the gradual increase of the included angle is referred to as the open state. Figure 5 and Figure 9 show an open state of the valve clamping device 100.

As shown in Figure 9, when the valve opens to form a large opening (that is, the opening between the leaflets 131 and 132) suitable for the valve clamping device 100 to pass through, the valve clamping device 100 can be sent into the left ventricle. In this embodiment, the valve clamping device 100 in the open state is sent into the left ventricle. In other embodiments, the valve clamping device 100 in the closed state can be sent into the left ventricle, and then the clamping member 121 is controlled to open gradually.

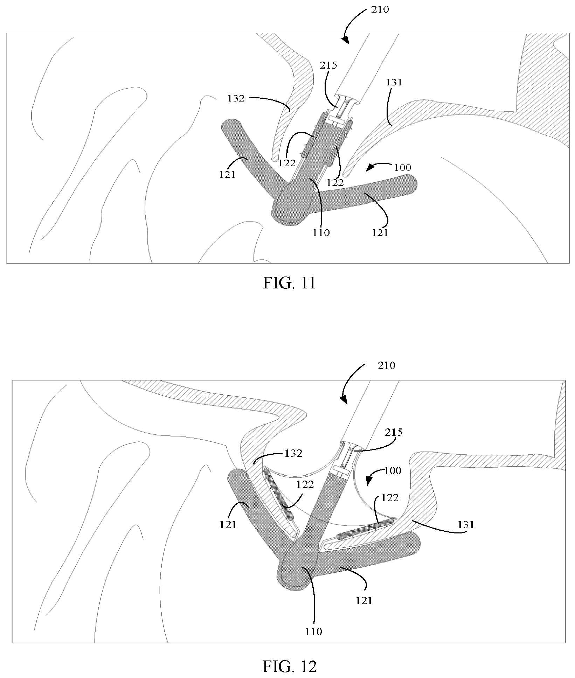

As shown in Figure 10, after the valve clamping device 100 is sent into the left ventricle, the operator can adjust the position of the valve clamping device 100 through images and judge whether the open state of the valve clamping device 100 is suitable for capturing the valve leaflets, that is, whether the clamping member 121 of the valve clamping device 100 is in the optimal capturing position. When the open state of the valve clamping device 100 is suitable for capturing the valve leaflets, the capturing member 122 can be controlled to open and move towards the clamping member 121 on the same side, so as to capture the valve leaflets between the capturing member 122 and the clamping member 121. For example, as shown in Figure 6 and Figure 11, when the included angle α between the clamping members 121 facing the proximal end of the valve clamping device 100 is an obtuse angle and the valve leaflets are located between the clamping members 121 and the capturing members 122, the valve clamping device 100 is controlled to approach one leaflet of the valve (referred to as the first leaflet for distinction). When the first leaflet is located between the capturing member 122 and the clamping member 121 on one side, the capturing member 122 on this side is controlled to move towards the clamping member 121 on the same side, so as to clamp the first leaflet between the capturing member 122 and the clamping member 121; then, in the same way, the valve clamping device 100 is controlled to approach the other leaflet of the valve (referred to as the second leaflet for distinction). When the second leaflet is located between the capturing member 122 and the clamping member 121 on the other side, the capturing member 122 on the other side is controlled to move towards the clamping member 121 on the same side, so as to clamp the second leaflet between the capturing member 122 and the clamping member 121; thus, as shown in Figure 12, the capture of the valve leaflets is completed. The first leaflet may be the leaflet 131, then the second leaflet is the leaflet 132, or the first leaflet may be the leaflet 132, then the second leaflet is the leaflet 131.

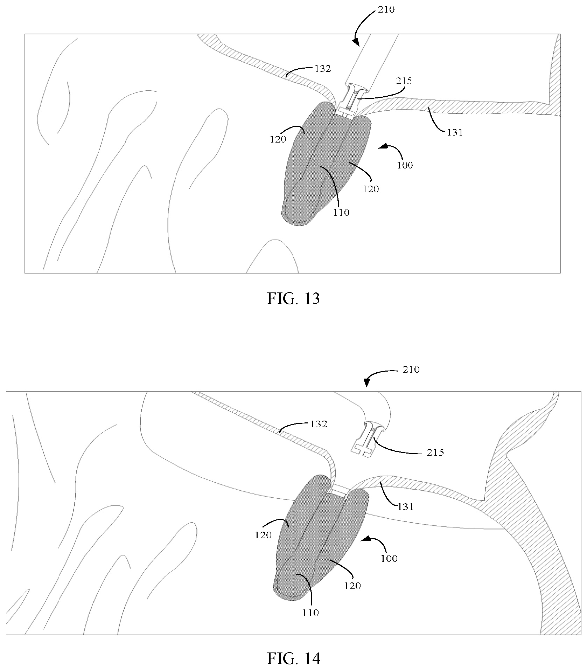

Then, the clamping member 121 is controlled to close towards the main body 110. Since the capturing member 122 is located between the clamping member 121 and the main body 110, the clamping member 121 can drive the capturing member 122 to close, so that the valve clamping device 100 returns to the closed state, as shown in Figure 13. The difference from the closed state during delivery is that the valve clamping device 100 clamps different valve leaflets at this time, so that the captured valve leaflets are opposed to each other to achieve the purpose of edge-to-edge repair. Finally, the valve clamping device 100 is controlled to disengage from the distal end of the catheter 210 and remain in the patient's body. The valve clamping device 100 and the catheter 210 are connected through a clutch system or mechanism (also referred to as a connection/disconnection system or mechanism). The clutch mechanism includes a connecting structure 116 at the proximal end of the valve clamping device 100 and a clutch device 215 at the distal end of the catheter 210. During delivery and operation, as shown in Figure 8 to Figure 13, the connecting structure 116 at the proximal end of the valve clamping device 100 and the clutch device 215 remain connected. After completing the capture and coaptation clamping of the valve leaflets, as shown in Figure 14, the connecting structure 116 is controlled to detach from the clutch device 215, so that the valve clamping device 100 in the closed state disengages from the distal end of the catheter 210 and remains in the patient's body. In the closed state, the clamping body 120 and the main body 110 have a very small included angle, so the main body 110 can be filled between the clamping bodies 120 to improve the clamping stability and reduce blood regurgitation.

The included angle between the clamping members 121 facing the proximal end of the valve clamping device 100 can be further increased. Thus, when the capture of the valve leaflets fails, the valve clamping device 100 can be controlled to the inverted state, as shown in Figure 7. At this time, the included angle β between the clamping members 121 facing the distal end of the valve clamping device 100 is an acute angle, which is conducive for the valve clamping device 100 to retreat from the ventricular side to the atrial side without winding around the chordae tendineae connected to the valve leaflets. In this way, it is convenient for the operator to control, and at the same time, the damage caused by the valve clamping device 100 to the tissues in the patient's body is reduced. After that, the position and state of the valve clamping device 100 can be readjusted, and the above steps of capturing and clamping the valve leaflets are repeated until the coaptation clamping of the valve leaflets is completed.

The valve clamping device 100 can be switched between the above states, and can be in any of the states shown in Figure 4 to Figure 7 above, or in any intermediate state between two adjacent states. All states except the closed state can be understood as an open state.

The valve clamping device and valve repair system according to the following embodiments of the present disclosure can be used not only for repairing the mitral valve and tricuspid valve, but also for repairing other similar valve structures.

In some aspects, the stability of the valve clamping device is of great significance for reducing the surgical difficulty and improving the repair effect. In view of this, some embodiments of the present disclosure provide a valve clamping device and a valve repair system, which have better stability when used by the operator, thereby reducing the operation difficulty for the operator.

In the embodiment of the present disclosure, the main body 110 adopts a segmented transmission configuration (referred to as a transmission assembly hereinafter) to drive the movement of the clamping member 121, so as to improve the stability of the valve clamping device 100. The segmented transmission assembly has a first section and a second section, and a transmission structure is arranged between the first section and the second section. The transmission structure provides transmission of axial motion, and at the same time, reduces (or prevents) the transmission of radial motion tendency to the second section, in other words, the transmission structure does not provide transmission of radial motion (it can be understood that the transmitted radial motion is negligible). In the embodiment of the present disclosure, radial motion can also be referred to as radial rotation.

In order to deliver and control the implant (for example, the valve clamping device 100 in the embodiment of the present disclosure) more stably, the control mechanism 2231 at the proximal end of the delivery system 200 usually makes the delivery rod 214 perform axial motion through rotation. At this time, both the radial motion and axial motion at the proximal end of the delivery rod 214 are transmitted to the main body 110 through the distal end of the delivery rod 214. After the main body 110 adopts the segmented structure, the radial motion is transmitted to the first section of the main body 110 and then isolated from the second section to a certain extent, which reduces the transmission of radial motion tendency to the second section. At this time, the second section drives the opening and closing motions of the clamping member 121, so that the motion of the clamping member 121 tends to change continuously and stably. During operation, the valve clamping device 100 has a more stable state, which facilitates the operator's control over the clamping member 121 and reduces the operation difficulty.

It can be seen that the above valve clamping device 100 can control the rotation pitch of the delivery rod 214 through the control mechanism 2231, so that the motion of the clamping member 121 tends to be continuous. The included angle between the clamping members 121 towards the proximal end of the valve clamping device 100 can present a continuous angle between 0° and 300°, which is conducive to the operator's control over the clamping member 121, improves the adaptability to different valve leaflet thicknesses, and broadens the applicable scenarios of transcatheter interventional edge-to-edge repair.

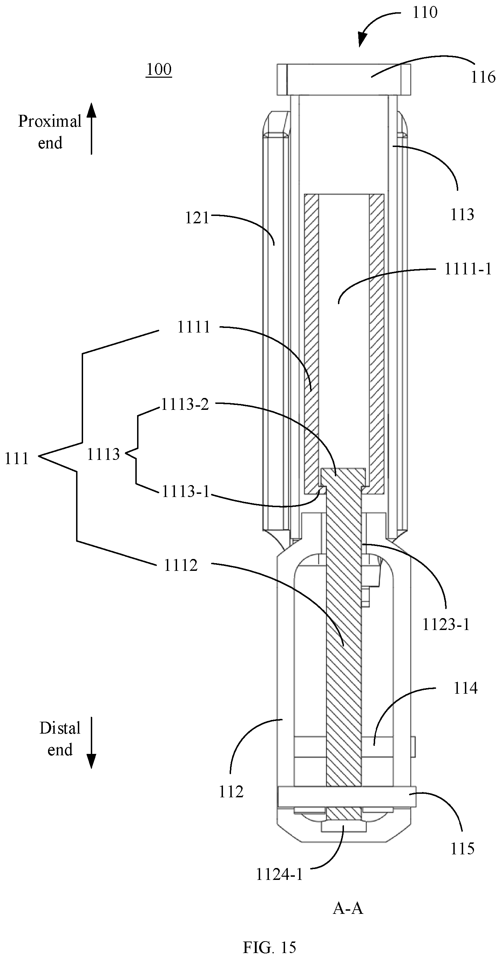

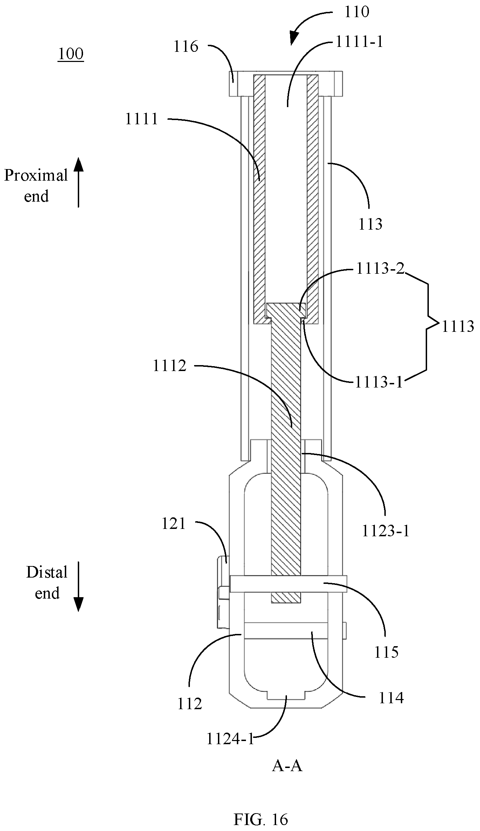

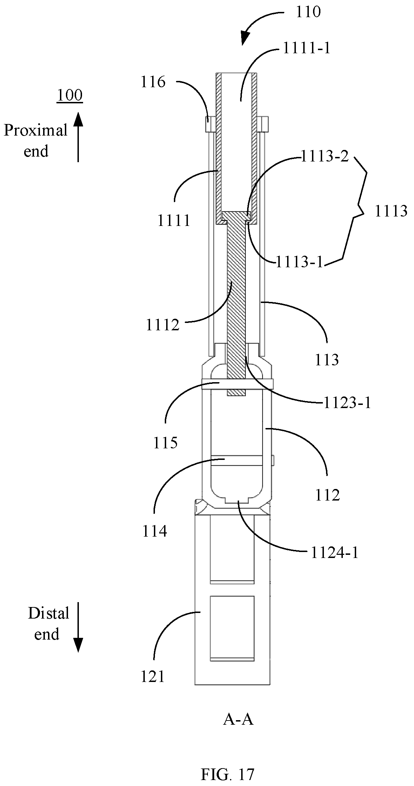

The following description is made with reference to the accompanying drawings. Please refer to Figure 15 to Figure 17, which show the structure of a main body 110 according to an embodiment of the present disclosure. Under the main body structure of this embodiment, Figure 15 is a cross-sectional view of the main body 110 of the valve clamping device shown in Figure 4 along the line A-A; Figure 16 is a cross-sectional view of the main body 110 of the valve clamping device shown in Figure 5 (or Figure 6) along the line A-A; Figure 17 is a cross-sectional view of the main body 110 of the valve clamping device shown in Figure 7 along the line A-A.

As shown in Figure 15 to Figure 17, the main body 110 includes a transmission assembly 111. The transmission assembly 111 includes a first transmission rod 1111 and a second transmission rod 1112. A transmission structure 1113 is arranged at the distal end of the first transmission rod 1111 and the proximal end of the second transmission rod 1112, and the transmission structure 1113 transmits axial motion between the first transmission rod 1111 and the second transmission rod 1112. The clamping member 121 of the clamping body 120 is hinged to the distal end of the second transmission rod 1112, so that the axial motion of the second transmission rod 1112 drives the opening and closing motions of the clamping member 121, so as to change the included angle between the clamping member 121 and the main body 110.

The transmission structure 1113 transmitting axial motion between the first transmission rod 1111 and the second transmission rod 1112 means that the transmission structure 1113 transmits the axial motion of the first transmission rod 1111 to the second transmission rod 1112, and/or the transmission structure 1113 transmits the axial motion of the second transmission rod 1112 to the first transmission rod 1111. While the transmission structure 1113 transmits axial motion between the first transmission rod 1111 and the second transmission rod 1112, the degree of transmission of radial motion is negligible or non-existent. That is to say, through the arrangement of the transmission structure 1113, a certain isolation effect is exerted on the radial force acting on the first transmission rod 1111, so that the second transmission rod 1112 only performs axial motion. That is, when the driving force for controlling the valve clamping device 100 acts radially on the first transmission rod 1111, the transmission structure 1113 transmits the axial motion while preventing the transmission of radial motion tendency to the second transmission rod 1112.

Through the segmented design, the above embodiment makes the radial component of the driving force for controlling the valve clamping device act only on the first section, and uses the transmission structure to transmit axial motion while preventing (or reducing) the transmission of radial force to the second section. The clamping member is hinged on the second section, so that the second section, which only performs axial motion, drives the opening and closing of the clamping member, making the motion of the clamping member tend to change continuously and stably. Thus, during operation, the valve clamping device has a more stable state, which facilitates the operator's control over the clamping member and reduces the operation difficulty.

Further, compared with the prior art in which a telescopic rod is arranged, and a connecting rod hinged to the clamping member is arranged on the outer wall of the telescopic rod, so that the telescopic rod can drive the clamping member to move through the connecting rod when the telescopic rod extends and retracts, the structural arrangement of the embodiment of the present disclosure is simpler, the factors leading to the unstable structure of the valve clamping device are reduced, and the stability of the valve clamping device can be improved to a certain extent.

In one embodiment, the control mechanism 2231 of the delivery system 200 for controlling the state of the valve clamping device 100 provides driving force to the valve clamping device 100 through the delivery rod 214. The driving force can be generated by clockwise or counterclockwise radial rotation. The driving force acts on the first transmission rod 1111, and in the first driving form (for example, clockwise or counterclockwise), the driving force drives the first transmission rod 1111 to perform radial motion and axial motion, the transmission structure 1113 transmits axial motion between the first transmission rod 1111 and the second transmission rod 1112, and reduces the transmission of radial motion, so that the second transmission rod 1112 performs axial motion. In the second driving form (for example, counterclockwise or clockwise), the driving force drives the first transmission rod 111 to perform radial motion and drives the second transmission rod 1112 to perform axial motion, the transmission structure 1113 transmits axial motion between the first transmission rod 1111 and the second transmission rod 1112, and reduces the transmission of radial motion, so that the second transmission rod 1112 only performs axial motion under the action of the driving force.

It can be seen that when the first transmission rod 1111 or the second transmission rod 1112 performs axial motion, the axial force generated between the first transmission rod 1111 and the second transmission rod 1112 by the transmission structure 1113 is sufficient to drive the axial motion of the second transmission rod 1112 or the first transmission rod 1111; when the first transmission rod 1111 performs radial motion, the radial force generated between the first transmission rod 1111 and the second transmission rod 1112 by the transmission structure 1113 is insufficient to drive the radial motion of the second transmission rod 1112.

The transmission system of the valve clamping device with the segmented main body can be understood as including a proximal transmission subsystem and a distal transmission subsystem, and the proximal transmission subsystem and the distal transmission subsystem are axially limited to transmit axial motion, and are not radially limited to reduce the transmission tendency of radial motion to the distal transmission subsystem.

The above transmission structure 1113 can be realized by an axial limiting structure, which has no radial limit, therefore, the radial friction force is small, which is insufficient to transmit radial motion.

The axial limiting structure includes a first limiting structure and a second limiting structure that can limit each other, which are configured to transmit axial motion while reducing the transmission tendency of radial motion. When the first transmission rod 1111 drives the first limiting structure to perform radial motion, the radial motion is not transmitted to the second transmission rod 1112. Such an arrangement has a simple and effective structure, and is conducive to processing and assembly.

In one embodiment, the transmission structure 1113 includes a first limiting structure 1113-1 and a second limiting structure 1113-2. The first limiting structure 1113-1 is arranged at the distal end of the first transmission rod 1111, the second limiting structure 1113-2 is arranged at the proximal end of the second transmission rod 1112, and the first limiting structure 1113-1 and the second limiting structure 1113-2 axially abut against each other when the first transmission rod 1111 or the second transmission rod 1112 moves axially.

The first limiting structure 1113-1 and the second limiting structure 1113-2 can limit each other, so that the first transmission rod 1111 and the second transmission rod 1112 remain connected during axial motion. Thus, axial motion can be transmitted between the first transmission rod 1111 and the second transmission rod 1112.

In one embodiment, the first limiting structure 1113-1 is integrally formed with the first transmission rod 1111, and the second limiting structure 1113-2 is integrally formed with the second transmission rod 1112. Thus, the valve clamping device 100 has a simple connection design, better stability, and lower cost.

In other embodiments, a non-integrally formed design can also be adopted. For example, the first limiting structure 1113-1 is integrally formed with the first transmission rod 1111, and the second limiting structure 1113-2 is fixedly connected to the second transmission rod 1112; or, the first limiting structure 1113-1 is fixedly connected to the first transmission rod 1111, and the second limiting structure 1113-2 is integrally formed with the second transmission rod 1112; or, the first limiting structure 1113-1 is fixedly connected to the first transmission rod 1111, and the second limiting structure 1113-2 is fixedly connected to the second transmission rod 1112.

Further, in order to reduce the difficulty of integral forming, the first limiting structure 1113-1 is formed at the distal end of the first transmission rod 1111 and has an inwardly extending extension part, so that the size of the opening of the first transmission rod 1111 towards the distal end of the valve clamping device 100 is smaller than the size of the inner chamber (or inner cavity) of the first transmission rod 1111. The second limiting structure 1113-2 is formed at the proximal end of the second transmission rod 1112 and has an outwardly extending extension part. Thus, after the proximal end of the second transmission rod 1112 enters the inner chamber of the first transmission rod 1111, the extension parts of the first limiting structure 1113-1 and the second limiting structure 1113-2 axially abut against each other, so that when the second transmission rod 1112 performs axial motion towards the distal end of valve clamping device 100, it drives the first transmission rod 1111 to perform axial motion. The first transmission rod 1111 and the second transmission rod 1112 are independent components. Through the design of the limiting structure, the radial friction force between the first transmission rod 1111 and the second transmission rod 1112 is insufficient to drive the radial motion of the second transmission rod 1112, thereby reducing the transmission tendency of the radial motion of the first transmission rod 1111 to the second transmission rod 1112.

The axial abutment between the first limiting structure 1113-1 and the second limiting structure 1113-2 limits the relative movement between the first transmission rod 1111 and the second transmission rod 1112 in one axial direction. For example, in Figure 15 to Figure 17, the movement of the first transmission rod 1111 relative to the second transmission rod 1112 towards the proximal end of the valve clamping device 100 is limited, and the movement of the second transmission rod 1112 relative to the first transmission rod 1111 towards the distal end of the valve clamping device 100 is limited. Thus, when the first transmission rod 1111 moves towards the proximal end of the valve clamping device 100, it can drive the second transmission rod 1112 to move towards the proximal end of the valve clamping device 100, and when the second transmission rod 1112 moves towards the distal end of the valve clamping device 100, it can drive the first transmission rod 1111 to move towards the distal end of the valve clamping device 100. This limiting method has a relatively simple structure, is easy to process, and has low cost.

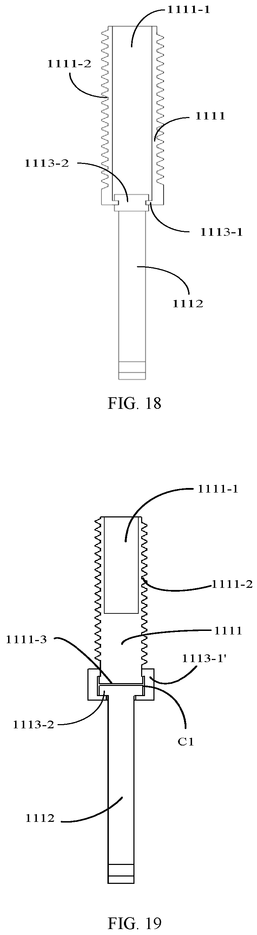

In other embodiments, the axial abutment between the first limiting structure 1113-1 and the second limiting structure 1113-2 limits the relative movement between the first transmission rod 1111 and the second transmission rod 1112 in two axial directions. One implementation is shown in Figure 18: the second limiting structure 1113-2 has a concave side surface, the first limiting structure 1113-1 has an extension part extending towards the interior of the first transmission rod 1111, and the extension part is configured to be located in the groove of the concave side surface. The groove is arranged around the circumference of the second limiting structure 1113-2, which is more conducive for the first transmission rod 1111 to perform radial motion relative to the second transmission rod 1112 without transmitting radial motion to the second transmission rod 1112. Optionally, the first limiting structure 1113-1 is integrally formed with the first transmission rod 1111, and the second limiting structure 1113-2 is fixedly connected to the second transmission rod 1112 or integrally formed with the second transmission rod 1112.

The above two-way limiting structure can bring the following benefits:

1. The axial motion of the second transmission rod 1112 towards the distal end of the valve clamping device 100 can be driven by the movement of the first limiting structure 1113-1 towards the distal end of the valve clamping device 100, without the abutment and push of the distal end point of the delivery rod. The length of the distal part of the delivery rod extending into the inner chamber of the first transmission rod 1111 can be shortened, which is conducive to the manipulation of the delivery rod.

2. The abutment area between the first limiting structure 1113-1 and the second limiting structure 1113-2 is small, which is conducive to reducing the transmission tendency of radial motion.

In some embodiments, the limiting structure can be arranged on the transmission rod as a limiting member independent of the transmission rod. For example, the first limiting structure can be a limiting member independent of the first transmission rod, or the second limiting structure can be a limiting member independent of the second transmission rod; or, both the first limiting structure and the second limiting structure are set as limiting members independent of the first transmission rod and the second transmission rod.

In some embodiments, the first limiting structure is a limiting member arranged at the distal end of the first transmission rod 1111, the limiting member has a chamber, the chamber has an opening towards the distal end of the valve clamping device 100, the second transmission rod 1112 can pass through the opening, the second limiting structure at the proximal end of the second transmission rod 1112 is accommodated in the chamber, and the maximum size of the second limiting structure is larger than the size of the opening. Or, the second limiting structure is a limiting member arranged at the proximal end of the second transmission rod 1112, the limiting member has a chamber, the chamber has an opening towards the proximal end of the valve clamping device 100, the distal end of the first transmission rod 1111 can pass through the opening, the first limiting structure at the distal end of the first transmission rod 1111 is accommodated in the chamber, and the maximum size of the first limiting structure is larger than the size of the opening towards the proximal end of the valve clamping device 100. The introduction of the limiting member brings a more flexible arrangement to the transmission structure, and each arrangement has its own advantages. Optionally, the limiting member can also be called a hub.

Several implementations are described below with reference to the accompanying drawings:

One implementation is shown in Figure 19. The first limiting structure is a limiting member 1113-1' arranged at the distal end of the first transmission rod 1111. For example, the proximal end of the limiting member 1113-1' is fixedly connected to the first transmission rod 1111 and is located near the distal end of the first transmission rod 1111. The limiting member 1113-1' has a chamber C1, the chamber C1 has an opening towards the proximal end of the valve clamping device 100, so that the distal end 1111-3 of the first transmission rod 1111 extends into the chamber C1 from the proximal end of the limiting member 1113-1'; the chamber C1 also has an opening towards the distal end of the valve clamping device 100, so that the proximal end of the second transmission rod 1112 extends into the chamber C1 from the distal end of the limiting member 1113-1', and the proximal end of the second transmission rod 1112 is provided with a second limiting structure 1113-2, which is accommodated in the chamber C1.

During axial transmission towards the distal end of the valve clamping device 100 (or during axial motion towards the distal end), the distal end 1111-3 of the first transmission rod 1111 abuts against the second limiting structure 1113-2. The opening of the chamber C1 towards the distal end of the valve clamping device 100 only allows (or just allows) the second transmission rod 1112 to pass through, and the maximum size of the second limiting structure 1113-2 is larger than the size of the opening towards the distal end of the valve clamping device 100. Therefore, during axial transmission towards the proximal end of the valve clamping device 100 (or during axial motion towards the proximal end), the distal end of the limiting member 1113-1' abuts against the second limiting structure 1113-2. In addition, the limiting member 1113-1' can rotate relative to the second transmission rod 1112.

Through the above structure, the proximal transmission subsystem where the first transmission rod 1111 is located becomes the main transmission subsystem, and the distal transmission subsystem where the second transmission rod 1112 is located becomes the passive transmission subsystem, which has the following benefits for the performance of the valve clamping device:

1. The axial length of the inner chamber 1111-1 of the first transmission rod 1111 can be shortened. Correspondingly, the length of the distal part of the delivery rod extending into the inner chamber 1111-1 of the first transmission rod 1111 is shortened. The shortened delivery rod is more conducive to the transmission of torque acting on the control mechanism at the proximal end of the delivery rod, and has better manipulation performance.

2. In the two axial movement directions, the distal part of the delivery rod only acts on the first transmission rod 1111, and the axial movement is realized through rotation, which reduces the number of components interacting with the distal end of the delivery rod, and is conducive to the performance stability of the valve clamping device 100.

3. The distal end 1111-3 of the first transmission rod abuts against the second limiting structure 1113-2 at the proximal end of the second transmission rod, so that the power transmission efficiency is higher when the transmission system moves axially towards the distal end of the valve clamping device 100.

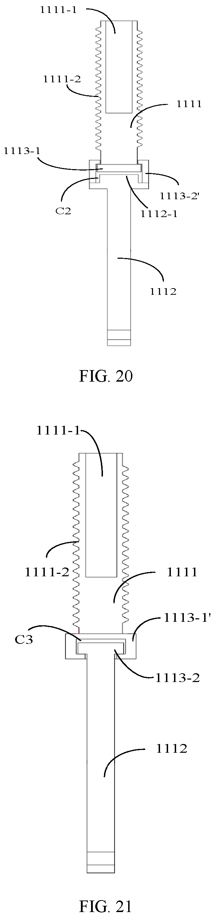

Another implementation is shown in Figure 20. The second limiting structure is a limiting member 1113-2' arranged at the proximal end of the second transmission rod 1112. For example, the distal end of the limiting member 1113-2' is fixedly connected to the second transmission rod 1112 and is located near the proximal end of the second transmission rod 1112. The limiting member 1113-2' has a chamber C2, the chamber C2 has an opening towards the proximal end of the valve clamping device 100, so that the distal end of the first transmission rod 1111 extends into the chamber C2 from the proximal end of the limiting member 1113-2', and the distal end of the first transmission rod 1111 is provided with a first limiting structure 1113-1, which is accommodated in the chamber C2; the chamber C2 also has an opening towards the distal end of the valve clamping device 100, so that the proximal end 1112-1 of the second transmission rod 1112 extends into the chamber C2 from the distal end of the limiting member 1113-2'.

During axial transmission towards the distal end of the valve clamping device 100 (or during axial motion towards the distal end), the first limiting structure 1113-1 at the distal end of the first transmission rod 1111 abuts against the proximal end 1112-1 of the second transmission rod 1112. The opening of the chamber C2 towards the proximal end of the valve clamping device 100 only allows the first transmission rod 1111 to pass through, and the maximum size of the first limiting structure 1113-1 is larger than the size of the opening towards the proximal end of the valve clamping device 100. Therefore, during axial transmission towards the proximal end of the valve clamping device 100 (or during axial motion towards the proximal end), the proximal end of the limiting member 1113-2' abuts against the first limiting structure 1113-1. In addition, the first transmission rod 1111 can rotate relative to the limiting member 1113-2'.

The above structure also makes the proximal transmission subsystem where the first transmission rod is located become the main transmission subsystem, and the distal transmission subsystem where the second transmission rod is located become the passive transmission subsystem, which has similar benefits for the performance of the valve clamping device 100 as the structure shown in Figure 19.

Yet another implementation is shown in Figure 21. The first limiting structure is a limiting member 1113-1' arranged at the distal end of the first transmission rod 1111. The difference from the implementation shown in Figure 19 is that the proximal end of the limiting member 1113-1' is not provided with an opening. The second limiting structure 1113-2 at the proximal end of the second transmission rod 1112 is accommodated in the chamber C3.

During axial transmission towards the distal end of the valve clamping device 100 (or during axial motion towards the distal end), the proximal inner wall of the limiting member 1113-1' abuts against the second limiting structure 1113-2. The opening of the chamber C3 towards the distal end of the valve clamping device 100 only allows the second transmission rod 1112 to pass through, and the maximum size of the second limiting structure 1113-2 is larger than the size of the opening towards the distal end of the valve clamping device 100. Therefore, during axial transmission towards the proximal end of the valve clamping device 100 (or during axial motion towards the proximal end), the distal end of the limiting member 1113-1' abuts against the second limiting structure 1113-2. In addition, the limiting member 1113-1' can rotate relative to the second transmission rod 1112.

The above structure also makes the proximal transmission subsystem where the first transmission rod is located become the main transmission subsystem, and the distal transmission subsystem where the second transmission rod is located become the passive transmission subsystem, which has similar benefits for the performance of the valve clamping device 100 as the structure shown in Figure 19.

In addition, the axial length of the limiting member 1113-1' can be further shortened, and thus the axial length of the valve clamping device 100 can be further shortened, which is more conducive to the transcatheter delivery of the valve clamping device 100 and the implantation operation in organs (for example, the heart).

The first limiting structure or the second limiting structure in each of the above embodiments can be set to be spherical or ellipsoidal, that is, the cross-section is circular or elliptical. When the second limiting structure is spherical or ellipsoidal, the first limiting structure can be provided with an accommodating chamber for accommodating the second limiting structure, and the accommodating chamber allows the second transmission rod to pass through but not the second limiting structure, that is, the maximum size of the second limiting structure is larger than the opening of the accommodating chamber. When the first limiting structure is spherical or ellipsoidal, the second limiting structure can be provided with an accommodating chamber for accommodating the first limiting structure, and the accommodating chamber allows the first transmission rod to pass through but not the first limiting structure, that is, the maximum size of the first limiting structure is larger than the opening of the accommodating chamber.

With such an arrangement, the abutment area between the components of the transmission structure at the distal end of the first transmission rod 1111 and the proximal end of the second transmission rod 1112 is small, which is conducive to further reducing the transmission tendency of radial motion.

Further, the inner wall of the accommodating chamber for accommodating the spherical or ellipsoidal limiting structure can be set as opposite inclined surfaces to better fit with the limiting structure accommodated therein. In this way, the contact area between the first limiting structure and the second limiting structure is small, and the contact surface has an angle with the radial direction, which is more helpful to reduce the transmission tendency of radial motion.

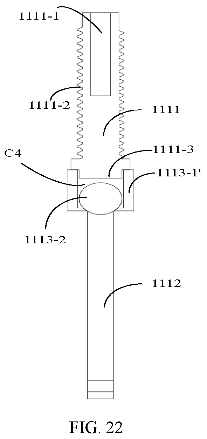

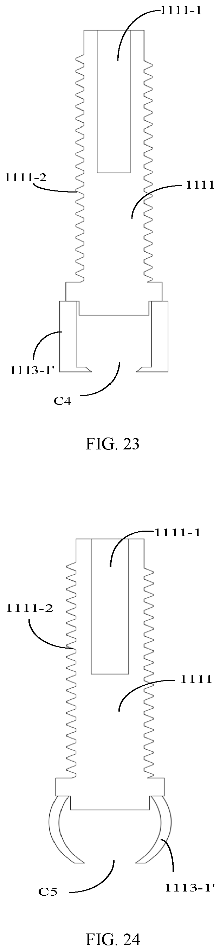

The solution of the above limiting structure can be applied to the embodiment provided with a limiting member, and the limiting structure accommodated in the chamber of the limiting member is set to be spherical or ellipsoidal. For example, as shown in Figure 22 and Figure 23, the second limiting structure 1113-2 arranged on the second transmission rod 1112 is spherical and is accommodated in the chamber C4 of the limiting member 1113-1'. Similarly, in other embodiments, the first limiting structure located in the chamber of the limiting member is spherical or ellipsoidal. Further, the inner wall of the chamber of the limiting member can be set as opposite inclined surfaces or arc surfaces to better fit with the limiting structure accommodated therein. For example, as shown in Figure 24, the inner wall of the chamber C5 of the limiting member 1113-1' has opposite arc surfaces.

The opening of the chamber in the above limiting member just allows the first transmission rod 1111 and/or the second transmission rod 1112 to pass through, so as to limit the radial shaking of the transmission rods and improve the stability of the valve clamping device 100.

In the above embodiments with a chamber, optionally, one or both of the first limiting structure and the second limiting structure have elasticity, that is, they are made of elastic materials. Thus, it is convenient to allow the limiting structure to extend into the chamber. For example, in the embodiments shown in Figure 19, Figure 21, and Figure 22, it is convenient to install the second limiting structure 1113-2, and in the embodiment shown in Figure 20, it is convenient to install the first limiting structure 1113-1. The elastic material is, for example, rubber, silica gel, or other materials with certain flexibility.

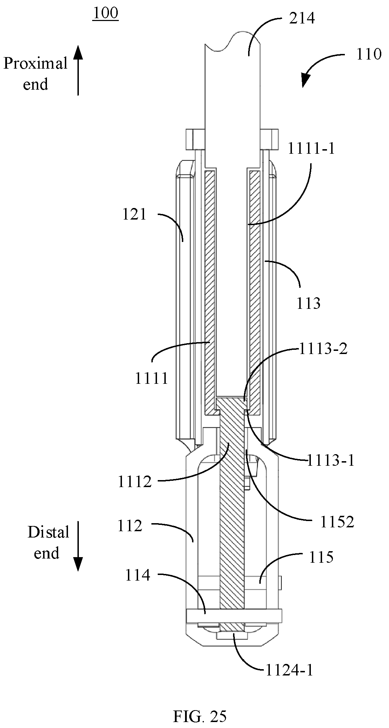

In some embodiments of the present disclosure, during the transcatheter delivery of the valve clamping device 100, the slender delivery rod 214 of the delivery system 200 is used to control the movement of the clamping member 121. Referring to Figure 25, the first transmission rod 1111 has an inner chamber 1111-1, the distal end of the delivery rod 214 of the delivery system 200 is suitable for extending into the inner chamber 1111-1 of the first transmission rod 1111, and the proximal end of the delivery rod 214 is connected to the control mechanism 2231. The operator can rotate the control mechanism 2231 at the proximal end of the delivery system 200 to make the delivery rod 214 perform axial motion.

In one embodiment, the outer side wall of the delivery rod 214 abuts against the inner chamber wall of the first transmission rod 1111, and the friction force between the delivery rod 214 and the first transmission rod 1111 drives the first transmission rod 1111 to move. When the delivery rod 214 drives the first transmission rod 1111 to move, it overcomes the resistance force. For example, for the hinged structure formed by the fixed pin, the moving pin and the corresponding track structure, the friction force between the delivery rod 214 and the first transmission rod 1111overcomes (exceeds) the friction force between the moving pin 115 on the support member 112 and the moving pin track 1125, and the friction force between the fixed pin 114 and the fixed pin track 1211-2; in addition, it overcomes the self-weight of the second transmission rod 1112. The movement of the first transmission rod 1111 includes axial motion and radial motion. The axial motion between the first transmission rod 1111 and the second transmission rod 1112 can be transmitted to change the state of the valve clamping device 100. After capturing and clamping the valve leaflets to achieve the purpose of edge-to-edge repair, the delivery rod 214 is separated from the first transmission rod 1111.

The shape arrangement of the distal part of the delivery rod 214 and the shape arrangement of the inner chamber 1111-1 of the first transmission rod 1111 can be matched with each other, so that the operation at the proximal end of the delivery rod 214 can be transmitted and act on the first transmission rod 1111. In one embodiment, neither the shape of the distal part of the delivery rod 214 nor the cross-sectional shape of the inner chamber 1111-1 of the first transmission rod 1111 is set to be circular, that is, non-circular. For example, the cross-section of the inner chamber 1111-1 of the first transmission rod 1111 is elliptical, rectangular, or triangular; for another example, the cross-section of the inner chamber 1111-1 of the first transmission rod 1111 is racetrack-shaped, and this cross-section includes two parallel sides and symmetrical arc sides connected between the two parallel sides. The cross-section of the distal end of the delivery rod 214 can be regular or irregular, as long as it is matched with the cross-section of the inner chamber 1111-1 to realize abutment. For example, the cross-section of the distal end of the delivery rod 214 is rectangular (including square) or other polygons.

Thus, when the delivery rod 214 performs radial motion, the distal end of the delivery rod 214 inside the first transmission rod 1111 can only rotate at a limited minimum angle, and its edge abuts against the inner chamber wall of the first transmission rod 1111, thereby driving the first transmission rod 1111 to perform axial motion and radial motion.

In other embodiments, the cross-section of the inner chamber 1111-1 of the first transmission rod 1111 can also be other regular or irregular shapes, and the delivery rod 214 can also be set to other regular or irregular shapes, as long as the delivery rod can abut against the inner chamber 1111-1 of the first transmission rod 1111 as soon as possible during radial motion.

In some embodiments, as shown in Figure 15 to Figure 18, the first transmission rod 1111 is provided with the inner chamber 1111-1 extending therethrough, and the delivery rod 214 can pass through the inner chamber 1111-1 of the first transmission rod 1111 to abut against the second transmission rod 1112. When the control mechanism 2231 controlling the delivery rod 214 rotates in the first direction (clockwise or counterclockwise) to push the delivery rod 214 to move axially towards the proximal end of the valve clamping device 100, the delivery rod 214 abuts against the inner wall of the inner chamber 1111-1 of the first transmission rod 1111, driving the first transmission rod 1111 to move towards the proximal end of the valve clamping device 100. The first transmission rod 1111 drives the first limiting structure 1113-1, and the first limiting structure 1113-1 drives the second limiting structure 1113-2, thereby driving the second transmission rod 1112 to move axially towards the proximal end of the valve clamping device 100; at this time, the valve clamping device 100 changes from the closed state to the open state. When the control mechanism 2231 controlling the delivery rod 214 rotates in the second direction (counterclockwise or clockwise) to push the delivery rod 214 to move axially towards the distal end of the valve clamping device 100, the distal end point of the delivery rod 214 abuts against the proximal end point of the second limiting structure 1113-2, pushing the second transmission rod 1112 to move towards the distal end of the valve clamping device 100. The second transmission rod 1112 drives the second limiting structure 1113-2, and the second limiting structure 1113-2 drives the first limiting structure 1113-1, thereby driving the first transmission rod 1111 to move axially towards the distal end of the valve clamping device 100; at this time, the valve clamping device 100 can change from the open state to the closed state. It can be seen that from the closed state to the open state (including the inverted state), the first limiting structure 1113-1 drives the second limiting structure 1113-2, and in the reverse movement, the second limiting structure 1113-2 drives the first limiting structure 1113-1.

In some embodiments, as shown in Figure 19 to Figure 24, the inner chamber 1111-1 of the first transmission rod 1111 is shortened and does not extend through the entirety of the first transmission rod 1111. At this time, in this embodiment, the axial movement of the first transmission rod 1111 towards the distal end of the valve clamping device 100 drives the second transmission rod 1112 to move axially towards the distal end of the valve clamping device 100, without the need for the distal end of the delivery rod 214 to abut against the second transmission rod 1112 or the second limiting structure 1113-2 for pushing. Thus, the length of the distal end of the delivery rod 214 extending into the inner chamber 1111-1 of the first transmission rod 1111 can be reduced, and the shortened delivery rod is more conducive to the transmission of torque acting on the handle at the proximal end of the delivery rod, with better manipulation performance. The embodiment shown in Figure 18 can also be similarly improved.

Similar to the above embodiments, in some embodiments of the present disclosure, the main body 110 of the valve clamping device 100 may further include an attachment member 113. The first transmission rod 1111 is arranged in the attachment member 113. The attachment member 113 is, for example, an attachment member sleeve. The clamping member 121 and the capturing member 122 can be arranged along the circumference of the attachment member 113 through the support member 112. Taking a pair of clamping members 121 as an example, they are respectively located on two sides of the attachment member 113. Similarly, a pair of capturing members 122 is respectively located on two sides of the attachment member 113.

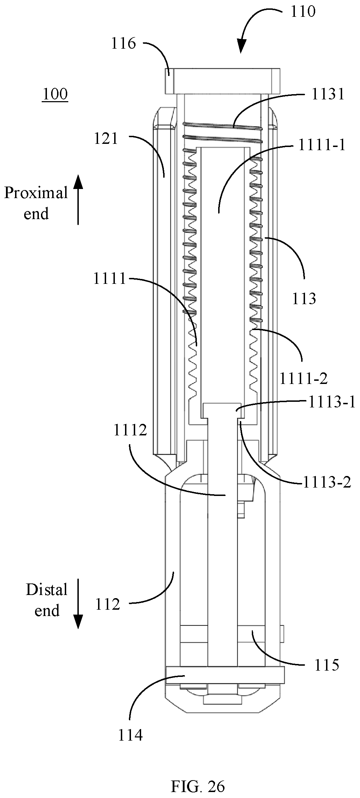

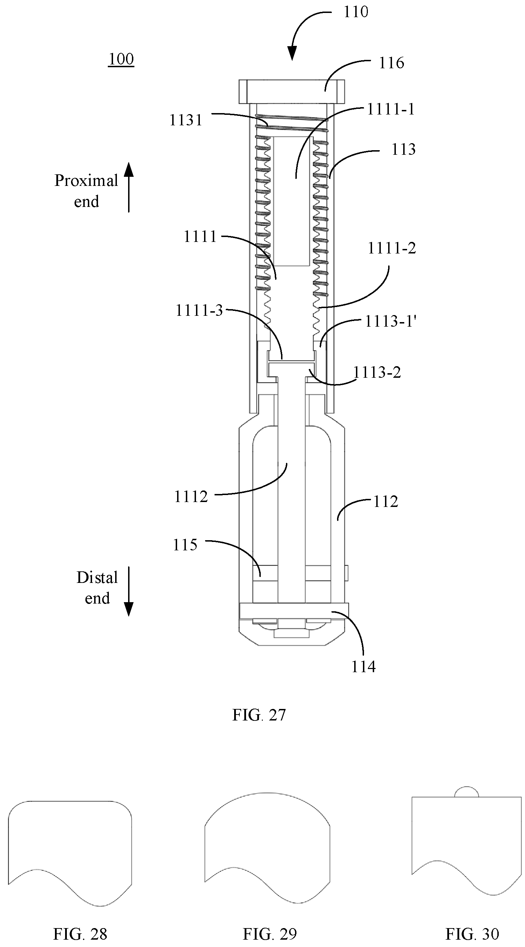

In some embodiments of the present disclosure, a locking structure (or locking system) can be provided in the valve clamping device 100, so that after capturing and clamping the valve leaflets, the valve clamping device 100 can be locked and maintained at the implantation position with a better effect. The locking structure can be realized by a thread structure. For example, referring to Figure 26 and Figure 27, in a specific embodiment, the inner side wall of the attachment member 113 is provided with an internal thread 1131, and the outer side wall of the first transmission rod 1111 is provided with an external thread 1111-2, and the internal thread 1131 matches the helix of the external thread 1111-2. Thus, when the first transmission rod 1111 rotates and moves axially through the thread, each step of the movement can be locked by the thread.

The present disclosure does not limit the length (or number) and position of the internal and external threads on the attachment member 113 and the first transmission rod 1111.

The arrangement of the above locking structure has the following benefits for the performance of the valve clamping device 100:
1. The opening angle of the clamping member 121 can be locked at any time, and there is no need to set a special control mechanism for the locking system in the delivery system. During use, the operator only needs to rotate the delivery rod to control the movement of the clamping member 121, and the state of the clamping member 121 is spontaneously locked. Continuing to rotate the delivery rod can realize further movement of the clamping member 121, and the operation steps are simple and effective.
2. The threaded connection between the first transmission rod and the attachment member makes the transmission system move axially more stably, which is conducive to the precise manipulation of the clamping member 121.

During the movement of the transmission assembly (or transmission system), it is expected that the axial motion can be transmitted efficiently between the two abutting surfaces, while the transmission of radial rotation is avoided as much as possible. Therefore, some settings can be made on the abutting surfaces to reduce the contact area between the abutting surfaces. For example, at least one of the abutting surfaces in the transmission assembly is provided with a convex structure, which includes, for example, a contact surface with an arc-shaped cross-section.

In different embodiments, the abutting surfaces are different. For example, in the embodiment shown in Figure 15 to Figure 17, a convex structure can be provided on at least one of the axially abutting surfaces of the first limiting structure 1113-1 and the second limiting structure 1113-2; optionally, the first limiting structure 1113-1 and the second transmission rod 1112 may or may not be in radial contact. When in contact, a convex structure can be provided on at least one of the radially abutting surfaces of the first limiting structure 1113-1 and the second transmission rod 1112. For another example, in the embodiment shown in Figure 18, a convex structure is provided on at least one of the axially abutting surfaces of the first limiting structure 1113-1 and the second limiting structure 1113-2; optionally, the first limiting structure 1113-1 and the second limiting structure 1113-2 may or may not be in radial contact. When in contact, a convex structure can be provided on at least one of the radially abutting surfaces. In the embodiment shown in Figure 19, a convex structure is provided on at least one of the axially abutting surfaces of the limiting member 1113-1' and the second limiting structure 1113-2, and the axially abutting surfaces of the distal end 1111-3 of the first transmission rod 1111 and the second limiting structure 1113-2. Similarly, if there are radially abutting surfaces, a convex structure can also be provided. In the embodiment shown in Figure 20, a convex structure is provided on at least one of the axially abutting surfaces of the limiting member 1113-2' and the first limiting structure 1113-1, and the axially abutting surfaces of the proximal end 1112-1 of the second transmission rod 1112 and the first limiting structure 1113-1. Similarly, if there are radially abutting surfaces, a convex structure can also be provided. In the embodiment shown in Figure 21, a convex structure is provided on at least one of the axially abutting surfaces of the limiting member 1113-1' and the second limiting structure 1113-2. Similarly, if there are radially abutting surfaces, a convex structure can also be provided. In each of the above embodiments, taking the embodiment shown in Figure 25 as an example, a convex structure is provided on at least one of the distal end surface of the delivery rod 214, the proximal end surface of the second transmission rod 1112, and the distal end surface of the inner chamber 1111-1 of the first transmission rod 1111.

The above convex structure can further reduce the transmission of radial motion, improve the transmission efficiency of axial motion, and thus further improve the stability of the clamping member 121 during operation.

The cross-section of any of the above abutting surfaces can be as shown in Figure 28, with arc-shaped chamfers on the edges to reduce the area of the abutting part and the transmission tendency of radial motion, and the reserved plane part in the cross-section ensures the motion transmission efficiency. In some embodiments, the area of the abutting surface can be further reduced by setting the cross-section to a partial spherical surface (for example, a hemispherical surface), as shown in Figure 29. In some embodiments, one or more spherical or partial spherical (for example, hemispherical) microstructures are arranged on the abutting surface to reduce the area of the abutting surface, as shown in Figure 30.

In other embodiments, other structural arrangements can also be used to reduce the abutting area between the limiting structure and the transmission rod, between the limiting structures, and between the transmission rods in the transmission assembly, as well as the abutting area between the delivery rod and the transmission rod, which will not be described one by one here, and all fall within the protection scope of the present disclosure.

Similar to the above embodiments, in some embodiments of the present disclosure, the main body 110 of the valve clamping device 100 may further include a support member 112 sleeved on the second transmission rod 1112. The support member 112 can be arranged on the attachment member 113 and directly connected to the attachment member 113, or the support member 112 can be indirectly connected to the attachment member 113 through the second transmission rod 1112.

The distal end of the second transmission rod 1112 is located in the support member 112 and is hinged with the clamping member 121. The axial motion of the second transmission rod 1112 drives the opening and closing motions of the clamping member 121, so as to change the included angle between the clamping member 121 and the main body 110. The connecting part of the clamping member 121 extends into the support member 112 and can be connected to the support member 112 through a pin.

The hinge connection between the clamping member 121 and the second transmission rod 1112 is a direct hinge connection or an indirect hinge connection, and the hinge form can be various. This embodiment does not limit the hinge form, as long as the axial motion of the second transmission rod 1112 can drive the clamping member 121 to perform opening and closing motions.

In some aspects, the state change and the overall miniaturization of the valve clamping device are of great significance for the delivery of the valve clamping device and the operability in a complex environment, which can reduce the operation difficulty for the operator and improve the repair effect.

In view of this, the embodiments of the present disclosure provide a valve clamping device and a valve repair system. The structure of the clamping member is optimized, and combined with the arrangement of the support member, the valve clamping device has a more compact structure. At the same time, the compact structure supports the state change of the clamping member to a larger opening angle, which is convenient for the operator to operate, reduces the operation difficulty of the valve clamping device in a more complex environment, reduces the possible injury or discomfort to the patient, and thus improves the effect of edge-to-edge repair.

The following description is made with reference to the accompanying drawings:

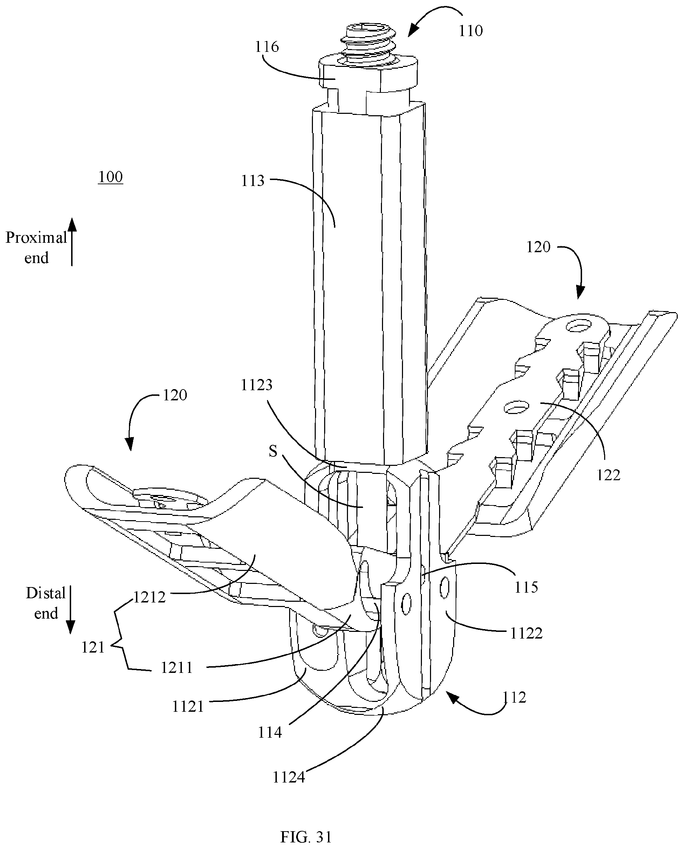

Please refer to Figure 31, which is a schematic perspective view of a valve clamping device according to an embodiment of the present disclosure. As shown in Figure 31, the main body 110 of the valve clamping device 100 includes a transmission member (not shown in the figure because it is located in the attachment member 113) and a support member 112. The transmission member can be arranged in the attachment member 113, and the support member 112 is located at the distal end of the main body 110 and connected to the attachment member 113. The attachment member 113 is, for example, an attachment member sleeve for accommodating and protecting the transmission member. In addition, a connecting structure 116 can be arranged on the attachment member 113 for connecting or detaching the valve clamping device 100 to or from the distal end of the catheter through the clutch device 215. In other embodiments, the main body 110 may not be provided with an attachment member, and the connecting structure is directly arranged on the transmission member to realize the connection or disconnection with the catheter; further, the support member can be sleeved on the distal end of the transmission member.

The transmission member can adopt the structure of the transmission assembly 111 in the above embodiments, or can adopt a non-two-segment structure or a telescopic structure, etc. This embodiment is not limited thereto, but combined with the structure of the transmission assembly 111 in the above embodiments, the stability of the valve clamping device can be further improved.

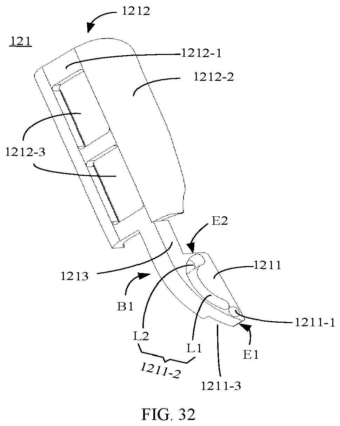

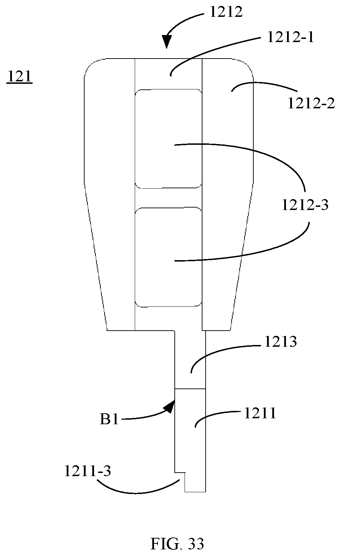

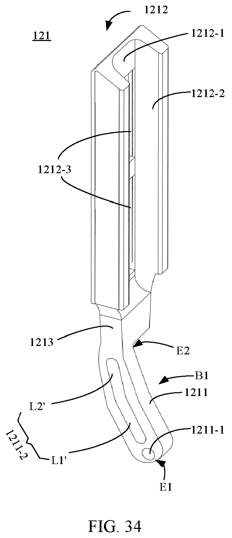

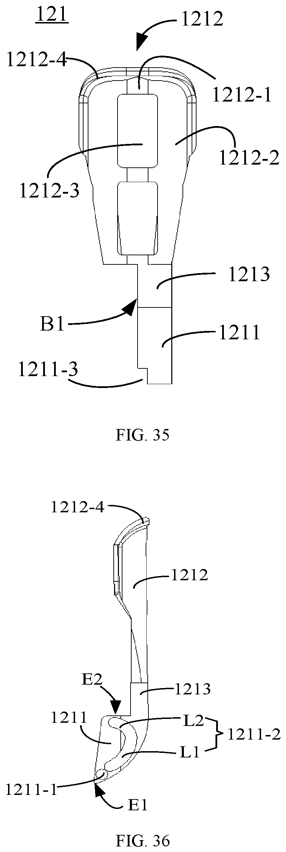

The support member 112 has an accommodating chamber S inside, the accommodating chamber S has an opening towards the outside, and is provided with a fixed pin 114 passing through the accommodating chamber S; the distal end of the transmission member is inserted into the accommodating chamber S of the support member 112, and moves axially relative to the support member 112 under the action of driving force. Please continue to refer to Figure 32 to Figure 36, which are schematic structural diagrams of different clamping members according to the embodiments of the present disclosure, respectively. Among them, Figure 32 is a schematic perspective view of a clamping member, and Figure 33 is a plan view of the clamping member shown in Figure 32; Figure 34 is a schematic perspective view of another clamping member; Figure 35 is a side view of yet another clamping member, and Figure 36 is another side view of the clamping member shown in Figure 35. The clamping member 121 includes a connecting part 1211 and a clamping part 1212, and the clamping part 1212 is connected to the connecting part 1211; the connecting part 1211 extends into the accommodating chamber S through the opening of the support member 112. The connecting part 1211 includes a moving pin hole 1211-1 and a fixed pin track 1211-2; the moving pin hole 1211-1 is located at the first end of the connecting part 1211, and is used to connect the farthest end of the connecting part 1211 away from the clamping part 1212 with the distal end of the transmission member through the moving pin 115 in the accommodating chamber S; the fixed pin track 1211-2 extends from the second end of the connecting part 1211 close to the clamping part 1212 to the first end to be adjacent to the moving pin hole 1211-1; the moving pin 115 passes through the moving pin hole 1211-1 and the distal end of the transmission member; thus, the axial motion of the transmission member drives the axial motion of the moving pin 115, the fixed pin 114 passes through the fixed pin track 1211-2, and the axial motion of the moving pin 115 changes the position of the fixed pin 114 in the fixed pin track 1211-2, so that the connecting part 1211 drives the clamping part 1212 to perform opening and closing motions along the opening in the direction limited by the fixed pin track 1211-2.

In the above embodiments, the moving pin hole 1211-1 is close to the end point of the connecting part 1211 (that is, the farthest end of the connecting part 1211 away from the clamping part 1212), and the fixed pin track 1211-2 is close to the tail end of the end point. Further, in conjunction with the arrangement of the support member 112, the moving pin 115 and the fixed pin 114, the clamping member 121 is subject to as little interference as possible during movement, and the envelope surface corresponding to the position passed by the connecting part 1211 supports a larger opening angle of the clamping part 1212 in the smallest possible range. In addition, this structural design is more compact, effectively utilizes the space of the connecting part 1211, so that the valve clamping device 100 has a smaller overall size, and at the same time supports a larger opening angle range of the valve clamping device 100.

In one embodiment, the fixed pin track 1211-2 adopts a two-segment design, wherein the first segment supports the change of the motion track of the connecting part 1211 of the clamping member 121 during the change of the valve clamping device 100 from the closed state to the open state when capturing the valve leaflets (as shown in Figure 5 and Figure 11, referred to as the first open state), and the second segment supports the change of the motion track of the connecting part 1211 of the clamping member 121 during the change of the valve clamping device 100 from the failed capture of the valve leaflets (i.e., the first open state) to the inverted state (as shown in Figure 7). That is, the fixed pin 114 is located at different positions in the first segment during the change of the valve clamping device 100 from the closed state to the first open state, and the fixed pin 114 is located at different positions in the second segment during the change of the valve clamping device 100 from the first open state to the inverted state; wherein the second segment enables the connecting part 1211 to support a faster opening speed of the clamping member 121 with a shorter motion track compared with the first segment, thus having better operability.

In one embodiment, the fixed pin track 1211-2 adopts an arc-shaped track design to reduce the resistance of the fixed pin 114 to the connecting part 1211 when the connecting part 1211 moves along the opening, making its movement smoother. At this time, the above first segment can be a first arc segment, and the second segment can be a second arc segment. Please continue to refer to Figure 32 and Figure 36. The fixed pin track 1211-2 includes a first arc segment L1 and a second arc segment L2. The first arc segment L1 is close to the first end E1, the second arc segment L2 is close to the second end E2, and the radian of the second arc segment L2 is greater than that of the first arc segment L1, or in other words, the radius of the first arc segment L1 is larger than that of the second arc segment L2, which shows that the bending of the second arc segment L2 is more obvious. Thus, the transmission member can drive the clamping member 121 to move in a larger range with a shorter axial movement distance, so that the valve clamping device 100 has a larger opening angle.

Further, the radial cross-sectional size of the connecting part 1211 of the clamping member 121 gradually decreases from the second end E2 to the first end E1. As shown in Figure 32 and Figure 36, the radial size of the connecting part 1211 gradually decreases in the direction away from the clamping part 1212, which is more suitable for the layout of the above fixed pin track 1211-2 and supports a more compact structural design of the clamping member 121. This embodiment shows an irregular fan-shaped (or referred to as fan-like) structure, the tail end is the end point of the fan shape, and the outer edge away from the opening of the support member 112 has an arc-shaped design, so that during the delivery of the valve clamping device, it has smaller resistance, is easier to deliver, and reduces the possible injury or discomfort to the patient. In other embodiments, other shape arrangements can also be employed, for example, an edge design with the same change trend as the fixed pin track 1211-2 on the side facing the opening.

In another embodiment, the fixed pin track 1211-2 may not adopt an arc-shaped track design. Please continue to refer to Figure 34, which adopts an intersecting straight segment design. The fixed pin track 1211-2 includes a first straight segment L1' and a second straight segment L2'. The first straight segment L1' is close to the first end E1, the second straight segment L2' is close to the second end E2, and the slope of the second straight segment L2' is greater than that of the first straight segment L1'. Thus, it can also be realized that the transmission member can drive the clamping member 121 to move in a larger range with a shorter axial movement distance, so that the valve clamping device 100 has a larger opening angle.

Please continue to refer to Figure 35 to Figure 37. The clamping part 1212 of the clamping member 121 is further provided with a hemming structure 1212-4, and the bending direction of the hemming structure 1212-4 is opposite to the clamping direction, so that during coaptation and clamping, the clamping member 121 is more closely attached to the main body 110 (for example, the attachment member 113), further improving the clamping effect of the valve clamping device. The hemming structure 1212-4 can also be referred to as a flange, which is arranged on all or part of the edge of the clamping part 1212. As shown in Figure 35 to Figure 37, in one embodiment, the hemming structure is arranged on the side edge and end edge with the largest width.

It can be seen that the independent design of the two-segment structure of the above fixed pin track 1211-2 can also support the valve clamping device to realize the state change of a larger opening angle with a more compact structure. Therefore, under this structural design, the position of the moving pin hole 1211-1 can be located in other places, for example, a position farther away from the first end E1 than in the above embodiments. However, adopting the position design in the above embodiments has a more compact structure, and has lower design requirements for the connecting part 1211 in the inner chamber of the support member 112, because this position makes the bottom end of the connecting part 1211 have less interference.

The structural design in the above embodiments enables the valve clamping device 100 to support a larger opening angle range with a more compact structure. For example, the opening and closing motions of the clamping member 121 makes the included angle between the clamping members 121 towards the proximal end of the valve clamping device 100 change between 0° and 300°.

The connection between the clamping part 1212 and the connecting part 1211 can be a direct connection or a connection through other parts. For example, the clamping part 1212 is connected to the connecting part 1211 through a support part 1213, so that through design, the capturing member 122 and the clamping member 121 can be more closely fitted when butted, improving the clamping performance of the valve clamping device, which will be described in the subsequent embodiments.

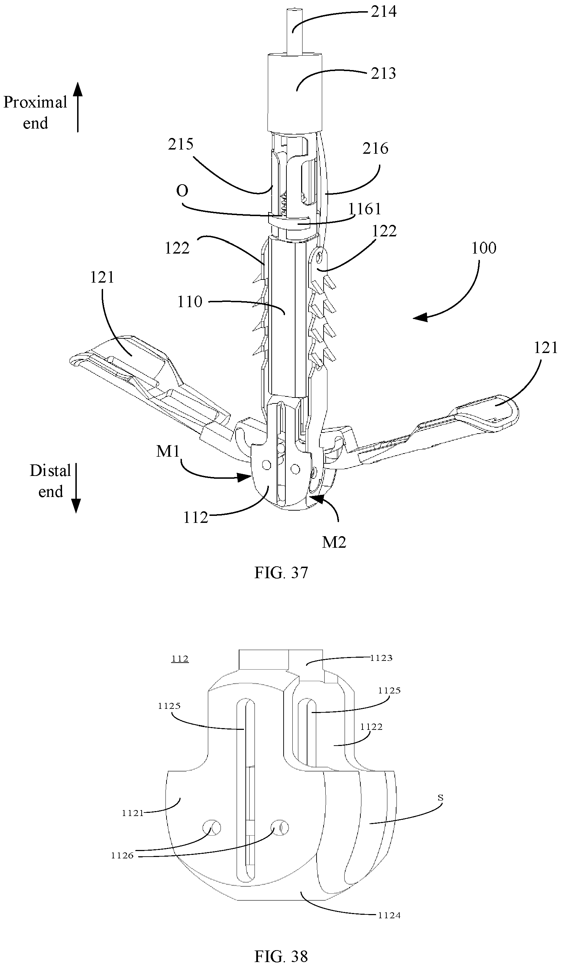

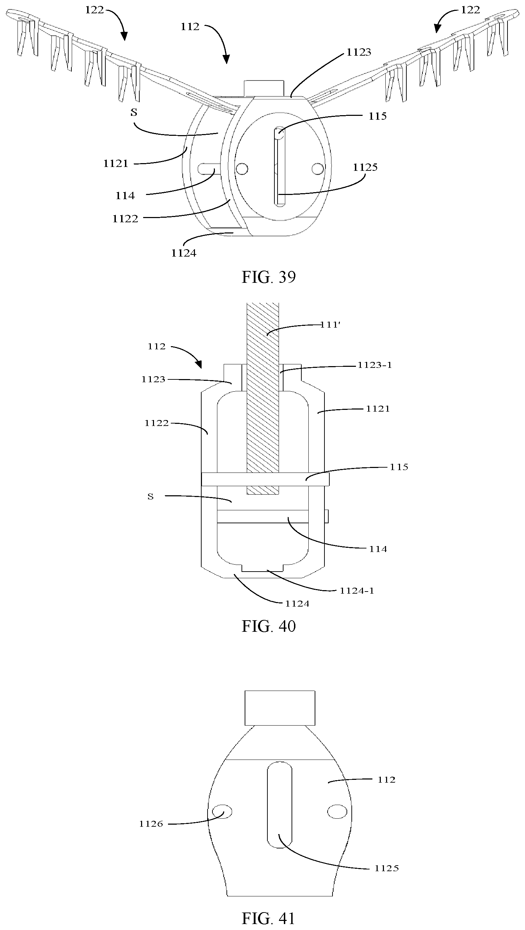

The structure of the support member 112 is described below with reference to the accompanying drawings:
Please continue to refer to Figure 31, and in conjunction with Figure 38 and Figure 39, which are schematic perspective views of different support members according to the embodiments of the present disclosure, respectively, wherein the capturing member 122 is installed as shown in Figure 39. The support member 112 includes a first support wall 1121 and a second support wall 1122 arranged oppositely. An accommodating chamber S is formed between the opposite surfaces of the first support wall 1121 and the second support wall 1122, and an opening is provided between the first support wall 1121 and the second support wall 1122. The support member 112 further includes a proximal connecting body 1123 (i.e., located at the proximal end of the support member 112) for connecting the first support wall 1121 and the second support wall 1122, that is, the first support wall 1121 and the second support wall 1122 are oppositely arranged on the proximal connecting body 1123. The fixed pin 114 is connected between the first support wall 1121 and the second support wall 1122; the distal end of the transmission member passes through the proximal connecting body 1123 and is located in the accommodating chamber S, and the axial motion of the transmission member changes its length in the accommodating chamber S.

Further, a moving pin track 1125 is provided on the first support wall 1121 and/or the second support wall 1122, which extends from the proximal end to the distal end of the support wall where it is located; the moving pin 115 moves axially along the moving pin track 1125 following the axial motion of the transmission member. When the moving pin track 1125 is provided on one support wall, the moving pin 115 is limited in its motion track by the moving pin track 1125 and the transmission member, that is, the moving pin 115 moves axially along the moving pin track 1125 following the axial motion of the transmission member; when the moving pin tracks 1125 are provided on both support walls, the two moving pin tracks 1125 are parallel to each other, and the moving pin 115 moves axially along the two moving pin tracks 1125 following the axial motion of the transmission member, which has a better limitation on the motion track of the moving pin 115 and improves the stability of the valve clamping device.

The moving pin track 1125 can be arranged in the form of hollowing out on the support wall; or in the form of a track groove arranged on the inner wall of the support wall, which is a groove on the inner wall but does not run through the entire support wall; or in the form of a track member arranged on the inner wall of the support wall. The hollow track can reduce the weight of the support member 112, and has the advantages of simple processing and high stability.

The fixed pin 114 is installed between the first support wall 1121 and the second support wall 1122, and its installation method is not limited, which can be pin joint, welding, clamping, etc. For example, as shown in Figure 38 and Figure 39, pin holes 1126 are provided on the first support wall 1121 and the second support wall 1122 for installing the fixed pin 114.

Further, the support member 112 further includes a distal connecting body 1124 (i.e., located at the distal end of the support member 112), and the first support wall 1121 and the second support wall 1122 are also oppositely arranged on the distal connecting body 1124, thus improving the stability of the support walls and further improving the stability of the valve clamping device. Optionally, as shown in Figure 40, the inner wall of the distal connecting body 1124 has a limiting groove 1124-1, and the axial motion of the distal end of the transmission member 111' towards the distal end of the valve clamping device in the accommodating chamber S is limited in the limiting groove 1124-1. Thus, when the distal end of the transmission member 111' moves to the distal end of the support member 112, it can be accommodated in the limiting groove 1124-1. At this time, the valve clamping device 100 is in a closed state, that is, when the clamping member 121 is clamped on the main body 110, the distal end of the transmission member 111' is accommodated in the limiting groove 1124-1. Since the limiting groove 1124-1 can limit the radial shaking of the transmission member 111', the stability of the valve clamping device 100 in the closed state can be further improved, and the repair effect can be enhanced.

The embodiment of the present disclosure can limit not only the distal end of the support member 112, but also the proximal end of the support member 112. At this time, the transmission member 111' passes through the proximal connecting body 1123 through the limiting channel 1123-1, that is, the proximal connecting body 1123 of the support member 112 has a limiting channel 1123-1, the transmission member 111' passes through the limiting channel, and the radial motion is limited by the limiting channel 1123-1. The size of the limiting channel 1123-1 just allows the transmission member 111' to pass through, which can limit the radial shaking of the transmission member 111', make the axial motion of the transmission member 111' more stable, and thus make the transmission more stable, which can effectively improve the stability of the valve clamping device 100 during use.

The present disclosure does not limit the cross-sectional shape of the support wall of the support member 112. In one embodiment, the support wall has a material reduction design at the proximal end and the distal end, that is, the cross-sectional size of the proximal end and the distal end is smaller than that of the middle section, but the cross-sectional size of the proximal end and the distal end can be the same or different (as shown in Figure 41). Among them, Figure 38, Figure 39, and Figure 41 respectively show different embodiments of the support member 112.

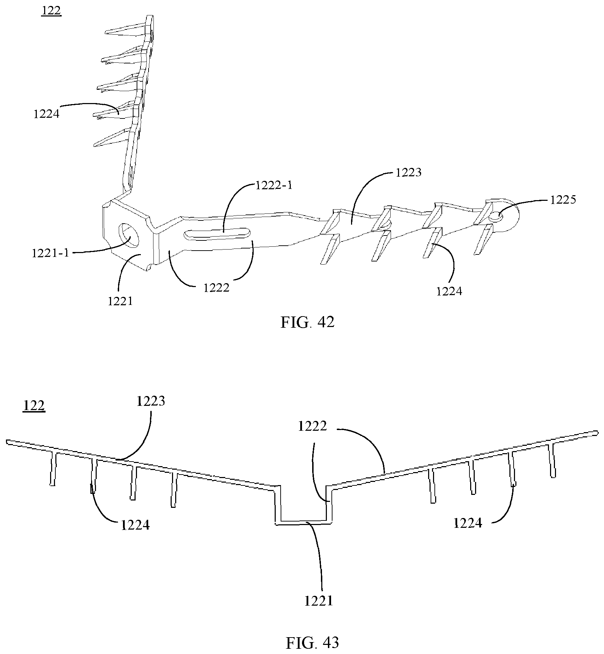

The capturing member is described below with reference to the accompanying drawings:
As shown in Figure 42 to Figure 46: the capturing member 122 includes a mounting part 1221, a bending part 1222, and a capturing part 1223. The bending part 1222 is connected between the mounting part 1221 and the capturing part 1223, so that the capturing part 1223 has a first included angle relative to the main body 110 (or the axial direction) in a natural state. The first included angle is greater than or equal to 60 degrees and less than or equal to 90 degrees, or the included angle between the capturing members 122 is in the range of 120 degrees to 180 degrees (including boundary values). Thus, the capturing member 122 has a shape suitable for capturing the valve leaflets in a natural state. After the capturing member 122 is released, it will quickly fit towards the clamping member 121, which is conducive to the capture of the valve leaflets.

In one embodiment, the capturing member 122 is provided with a connecting hole 1225 for connecting with a control mechanism for controlling the capturing member 122. For example, as shown in Figure 37, the control mechanism controls the state of the capturing member 122 through a control wire 216. The control wire 216 passes into the connecting hole 1225. When the capturing member 122 does not capture the valve leaflets, the control wire 216 is tightened, and the capturing member 122 is attached to the main body 110; when the capturing member 122 captures the valve leaflets, the control wire 216 is loosened, and the capturing member 122 quickly opens towards the clamping member 121 under no external force control (natural state), and is butted with the clamping member 121 to capture the valve leaflets.

Further, the bending part 1222 or the whole of the capturing member 122 is made of an elastic material, which can be an elastic alloy, for example, nitinol alloy. The elastic material facilitates the repeated opening and closing movements of the capturing member 122, and since the capturing member 122 has a first included angle with the main body 110 in a natural state, the capturing member 122 can quickly open towards the clamping member 121 to capture the valve leaflets. The whole capturing member 122 can be integrally formed of elastic material to reduce the production cost; or, the bending part 1222 of the capturing member 122 is made of elastic material, and the remaining all or part of the other parts of the capturing member 122 (for example, the mounting part) are made of rigid material to improve the connection rigidity.

Further, multiple friction elements 1224 are arranged on the capturing part 1223. The clamping member 121 is provided with hollows 1212-3. As shown in Figure 31, when the capturing member 122 is butted with the clamping member 121, the friction elements 1224 extend into the hollows 1212-3. Thus, not only can the weight of the clamping member 121 be reduced, but also the clamping effect can be improved, so that the valve clamping device 100 is easier to be controlled during operation and has a better clamping effect.

In one embodiment, at least one hollow 1222-1 is arranged on the bending part 1222 of the capturing member 122 to realize material reduction of the bending part 1222. Thus, when the capturing member 122 cooperates with the clamping member 121 to capture the valve leaflets and perform opening and closing movements, the bending part 1222 has sufficient toughness and low rigidity.

Optionally, the support member 112 can further support the installation of the capturing member 122, for example, it can support the capturing member 122 to be installed on the side end face or in the accommodating chamber S, which will be described in detail in the following embodiments.

In some aspects, the stability of the valve clamping device is of great significance for the effect of valve repair. In view of this, some embodiments of the present disclosure provide a valve clamping device and a valve repair system, which have better stability when used by the operator, thereby improving the effect of edge-to-edge repair.

In the embodiment of the present disclosure, a support member is arranged at the distal end of the valve clamping device, and the support member is used for supporting the clamping body. The support member has an accommodating chamber inside, and the accommodating chamber has an opening towards the outside, so that the connecting part of the clamping member extends into the accommodating chamber, and the position change in the accommodating chamber drives the clamping part to open and close relative to the main body. In addition, the capturing member includes a mounting part, a capturing part, and a bending part arranged between the mounting part and the capturing part. The mounting part is fixedly arranged on the support member to fixedly mount the capturing member on the support member. The bending part makes the capturing part open relative to the main body in a natural state (that is, has a first included angle). In the valve clamping device with this structure, the connecting part of the clamping member is movably arranged in the support member, and the mounting part of the capturing member is fixed on the support member. Thus, when driving the opening and closing of the clamping member, the state of the capturing member is more stable and will not move with the driving force, so that the stability of the valve clamping device is improved, and a better repair effect is achieved.

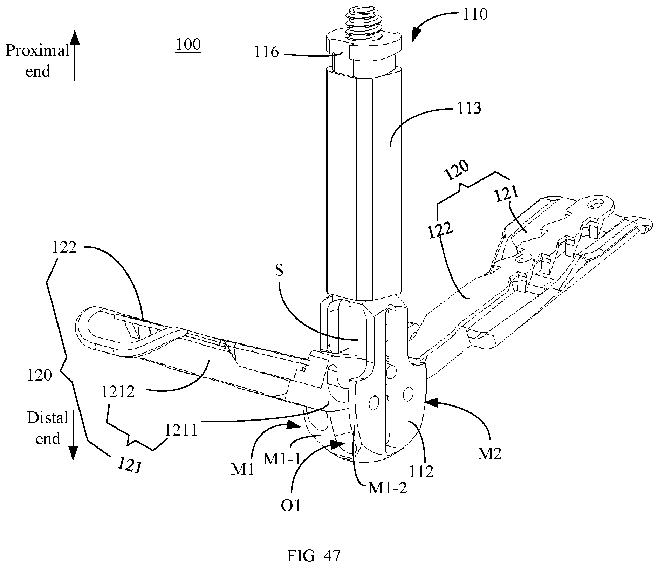

The following description is made with reference to the accompanying drawings. Please refer to Figure 47, which is a schematic perspective view of another valve clamping device according to an embodiment of the present disclosure. The valve clamping device 100 includes a main body 110 and a clamping body 120. The distal end of the main body 110 is provided with a support member 112 for supporting or mounting the clamping body 120; the clamping body 120 includes clamping members 121 and capturing members 122. The number of the clamping body 120 is multiple, that is, two or more, arranged along the circumference of the main body 110, and can perform opening and closing motions relative to the main body 110. For the convenience of description, two clamping bodies 120 are taken as an example for description here, and the implementation of more clamping bodies is similar thereto. In addition, the embodiment of Figure 31 can also be designed with a structure similar to this embodiment.

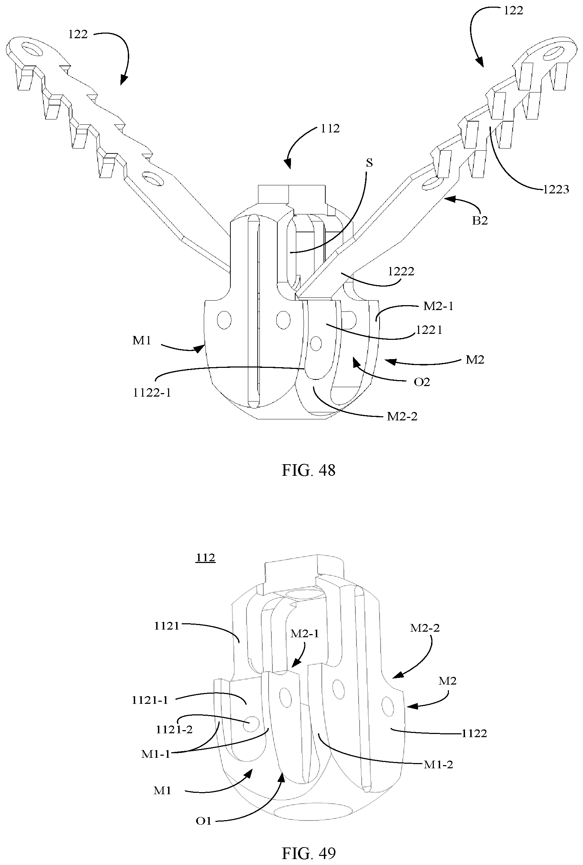

Please refer to Figure 48 in conjunction, which is a schematic perspective view of another perspective of the support member with the capturing member mounted thereon in the embodiment shown in Figure 47. To clearly reflect the structure of the support member, only the capturing member and the support member are illustrated in Figure 48. As shown in Figure 47 and Figure 48, the support member 112 has a first opening side M1 and a second opening side M2, and the support member 112 has an accommodating chamber S inside. The first opening side M1 has a first opening O1 that makes the accommodating chamber S open towards the outside, and the second opening side M2 has a second opening O2 that makes the accommodating chamber S open towards the outside.

Please continue to refer to Figure 47. The clamping member 121 includes a connecting part 1211 and a clamping part 1212. The connecting part 1211 extends into the accommodating chamber S through the opening of the support member 112 and is mounted in the accommodating chamber S. The position change of the connecting part 1211 in the accommodating chamber S drives the clamping part 1212 to open and close relative to the main body 110. For distinction, the clamping member 121 whose connecting part 1211 extends into the accommodating chamber S through the first opening O1 of the support member 112 is referred to as a first clamping member, and the clamping member 121 whose connecting part 1211 extends into the accommodating chamber S through the second opening O2 of the support member 112 is referred to as a second clamping member; that is, the clamping member 121 on the same side as the first opening side M1 of the support member 112 is the first clamping member, and the clamping member 121 on the same side as the second opening side M2 of the support member 112 is the second clamping member.

Please refer to Figure 48 in conjunction. The capturing member 122 includes a mounting part 1221, a bending part 1222, and a capturing part 1223. The mounting part 1221 is fixedly arranged on the support member 112, that is, the mounting part 1221 is used to fixedly mount the capturing member 122 on the support member 112. The bending part 1222 is connected between the mounting part 1221 and the capturing part 1223, so that the capturing part 1223 has a first included angle relative to the main body 110 in a natural state. For distinction, the capturing member located on the same side of the main body as the first clamping member is referred to as a first capturing member, and the capturing member located on the same side of the main body as the second clamping member is referred to as a second capturing member. The mounting part of the first capturing member is referred to as a first mounting part, the bending part is referred to as a first bending part, and the capturing part is referred to as a first capturing part; the mounting part of the second capturing member is referred to as a second mounting part, the bending part is referred to as a second bending part, and the capturing part is referred to as a second capturing part.

It can be seen that in the valve clamping device of the above embodiment, the connecting part of the clamping member is movably mounted in the accommodating chamber inside the support member, and the mounting part of the capturing member is fixed on the support member. Thus, the support member provides an installation space for the clamping member and a driving space for the connecting part during opening and closing motions, and provides a fixed installation position for the capturing member. Therefore, when driving the opening and closing of the clamping member, the state of the capturing member is more stable and will not move with the driving force, so that the stability of the valve clamping device is improved, and a better repair effect is achieved during use.

When installing the capturing member 122, the capturing part 1223 can be arranged between the main body 110 and the clamping member 121 on the same side. Thus, after the clamping member 121 and the capturing member 122 on the same side capture the valve leaflets, when the clamping member 121 is closed relative to the main body 110, it can drive the capturing member 122 on the same side to close together. Further, when the clamping member 121 is clamped on the main body 110, the capturing part 1223 of the capturing member 122 on the same side is clamped between the main body 110 and the clamping member 121.

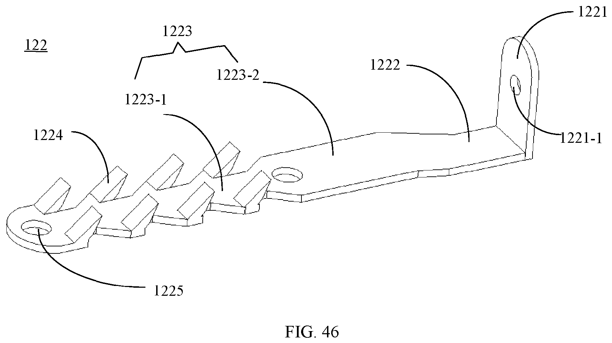

The structure of the capturing member is described below with reference to the accompanying drawings:
Please refer to Figure 42 to Figure 46, among which Figure 42 to Figure 45 show an integrally arranged capturing member structure; Figure 46 shows a separately arranged capturing member structure. The capturing member 122 includes a mounting part 1221, a bending part 1222, and a capturing part 1223. The mounting part 1221 is used to fixedly mount the capturing member 122 on the support member 112. The bending part 1222 is connected between the mounting part 1221 and the capturing part 1223, so that the capturing part 1223 has a first included angle relative to the main body 110 (or the axial direction) in a natural state. The first included angle is greater than or equal to 60 degrees and less than or equal to 90 degrees, or the included angle between the capturing members 122 is in the range of 120 degrees to 180 degrees (including boundary values). Thus, the capturing members 122 have a shape suitable for capturing the valve leaflets in a natural state. After the capturing member 122 is released, it will quickly fit towards the clamping member 121, which is conducive to the capture of the valve leaflets.

In one embodiment, the capturing member 122 is provided with a connecting hole 1225 for connecting with a control mechanism for controlling the capturing member 122. For example, as shown in Figure 37, the control mechanism controls the state of the capturing member 122 through a control wire 216. The control wire 216 extends into the connecting hole 1225. When the capturing member 122 does not capture the valve leaflets, the control wire 216 is tightened, and the capturing member 122 is attached to the main body 110; when the capturing member 122 captures the valve leaflets, the control wire 216 is loosened, and the capturing member 122 quickly opens towards the clamping member 121 under no external force control (natural state), and is butted with the clamping member 121 to capture the valve leaflets.

Further, the bending part 1222 or the whole of the capturing member 122 is made of an elastic material, which can be an elastic alloy, for example, nitinol alloy. The elastic material facilitates the repeated opening and closing movements of the capturing member 122, and since the capturing member 122 has a first included angle with the main body 110 in a natural state, the capturing member 122 can quickly open towards the clamping member 121 to capture the valve leaflets. The whole capturing member 122 can be integrally formed of elastic material to reduce the production cost; or, the bending part 1222 of the capturing member 122 is made of elastic material, and the remaining all or part of the other parts of the capturing member 122 (for example, the mounting part) are made of rigid material to improve the connection rigidity.

Further, multiple friction elements 1224 are arranged on the capturing part 1223. The clamping member 121 is provided with hollows 1212-3. As shown in Figure 31 and Figure 47, when the capturing member 122 is butted with the clamping member 121, the friction elements 1224 extend into the hollows 1212-3. Thus, not only can the weight of the clamping member 121 be reduced, but also the clamping effect can be improved, so that the valve clamping device 100 is easier to be controlled during operation and has a better clamping effect.

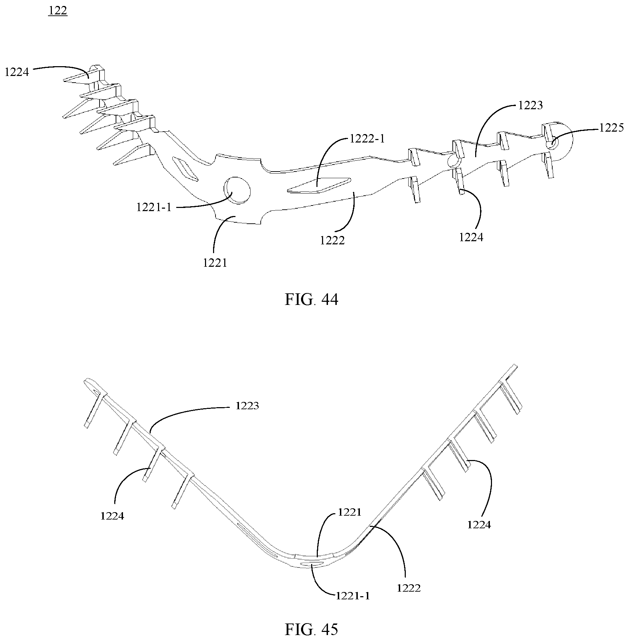

In one embodiment, as shown in Figure 42 and Figure 44, at least one hollow 1222-1 is arranged on the bending part 1222 of the capturing member 122 to realize material reduction of the bending part 1222. Thus, when the capturing member 122 cooperates with the clamping member 121 to capture the valve leaflets and perform opening and closing movements, the bending part 1222 has sufficient toughness and low rigidity.

In one embodiment, as shown in Figure 42 and Figure 44, the mounting parts 1221 of the capturing members 122 are integrally formed into a common mounting part, and have a through hole 1221-1 for the transmission member, which drives the opening and closing of the clamping member 121, to pass through. In the separately arranged capturing member structure, the arrangement of the through hole does not need to be considered, and the movement of the transmission member will not affect the capturing member, therefore, the stability of the valve clamping device is better. The present disclosure does not limit the shape of the common mounting part of the capturing member 122, which can be a regular shape or an irregular shape. An approximately square structure is shown in Figure 42 and Figure 44, with arc-shaped notches at four corners to facilitate clamping with the support member 112. In other embodiments, the mounting part can also be other polygons or irregular figures.

In some embodiments of the present disclosure, when the clamping member 121 and the capturing member 122 are butted, the side end face B2 of the capturing member 122 is in contact with the adjacent side end face B1 of the clamping member 121 (as shown in Figure 48 and Figure 32 to Figure 35). Thus, not only the capturing member 122 and the clamping member 121 have a smaller gap and better fit in the butting direction, so that the clamping member 121 and the capturing member 122 on the same side fit better, but also a more compact structural design is achieved in the direction perpendicular to the butting direction, realizing the design of smaller size of the valve clamping device 100. When the parts of the adjacent side end faces of the capturing member 122 and the clamping member 121 in contact are matched, the valve clamping device 100 has a compact structure, better clamping performance, and better stability of the overall structure.

By adopting the separate arrangement of the capturing members on different sides, the separately arranged capturing members are more conducive to the fitting design of the capturing member and the clamping member on the same side. The fitting problem between the capturing member and the clamping member can be solved through a simple installation position design, and a clearance space is provided between the capturing member and the clamping member, which can reduce the overlapping part between the clamping member and the capturing member in the butting direction (i.e., the opening and closing direction). Thus, when the capturing part of the capturing member and the clamping part of the clamping member are butted together (as shown in Figure 31 and Figure 47), the capturing member and the clamping member have a smaller gap and better fit in the butting direction, so that the clamping member and the capturing member on the same side fit better, thereby having better clamping performance.

Please continue to refer to Figure 47 to Figure 48. The capturing members 122 are separately arranged on different opening sides of the support member 112, for example, the first capturing member and the second capturing member are separately arranged on the first opening side M1 and the second opening side M2. This arrangement has a better fit between the capturing member 122 and the clamping member 121 during butting, and provides a simpler space for the accommodating chamber S, facilitating the position change of the connecting part 1211 of the clamping member 121. Thus, it is conducive to reducing the overall size of the valve clamping device 100 and supporting a wider range of opening angle changes.

Please refer to Figure 49 in conjunction, which is a schematic perspective view of another perspective of the support member in the embodiment shown in Figure 47. As shown in Figure 47 to Figure 49, the first opening side 1121 includes a side end face M1-1 and a side end face M1-2 (referred to as a first side end face and a second side end face respectively for distinction) located on two sides of the first opening O1; similarly, the second opening side 1122 includes a side end face M2-1 and a side end face M2-2 (referred to as a third side end face and a fourth side end face respectively for distinction) located on two sides of the second opening O2. The side end face M1-1 is opposite to the side end face M2-1, and the side end face M1-2 is opposite to the side end face M2-2; wherein, the first capturing member is mounted on the first side end face through its mounting part, and the second capturing member is mounted on the fourth side end face through its mounting part. It can be seen that in this structure, the capturing members are non-axisymmetrically mounted on two sides of the support member, but adopt a diagonal mounting method, so that the overall structure of the valve clamping device tends to be balanced and more stable. Thus, it is more conducive to the delivery and operation of the valve clamping device, and has a better repair effect during use.

In one embodiment, the thickness of the side end face where the capturing member is mounted can be increased. This thickened design structure can not only better accommodate the mounting part 1221 of the capturing member 122, but also the support member 112 has a supporting effect on the installation of the clamping member 121 in the accommodating chamber S. Therefore, the thickened design can better support the clamping member 121 and improve the overall stability of the valve clamping device 100. For example, the side end face M1-1 where the first capturing member is mounted and the side end face M2-2 where the second capturing member is mounted have a first thickness, the side end face M1-2 and the side end face M2-1 have a second thickness, and the first thickness is greater than the second thickness.

The mounting part 1221 of the capturing member 122 can be mounted on the side end face by means of pin joint, clamping, welding, interference fit, etc. The present disclosure does not limit the mounting method of the capturing member 122. In one embodiment, as shown in Figure 48 and Figure 49, the side end face M1-1 and the side end face M2-2 are respectively provided with an accommodating groove 1121-1 and an accommodating groove 1122-1, and the mounting part 1221 of the capturing member 122 is arranged in the accommodating groove and matches the accommodating groove. For example, the mounting part of the first capturing member is arranged in the accommodating groove 1121-1 of the side end face M1-1 and matches the accommodating groove 1121-1; the mounting part of the second capturing member is arranged in the accommodating groove 1122-1 of the side end face M2-2 and matches the accommodating groove 1122-1. Thus, the mounting part 1221 of the capturing member 122 is mounted in and matched with the accommodating groove, making its installation more stable. Further, the thickness of the mounting part 1221 matches the depth of the accommodating groove, so that the mounting part 1221 is flush with the outer edge of the side end face of the support member 112. Thus, the support member 112 has a smoother outer edge, so that the valve clamping device 100 can enter the patient's body more smoothly, which can effectively reduce the damage of the valve clamping device 100 to the patient or relieve the discomfort of the patient. In one embodiment, a pin joint hole (the pin joint hole 1121-2 in the accommodating groove 1121-1 is shown in the figure) is arranged in the accommodating groove, and a pin joint hole 1221-1 is also arranged on the mounting part 1221 of the capturing member 122, so as to pin the mounting part 1221 of the capturing member 122 in the accommodating groove.

Please continue to refer to Figure 49. The support member 112 includes a main end face and a side end face. The inner side faces of the main end face are arranged oppositely to form the accommodating chamber S of the support member 112, and the outer side faces are parallel to the inner side faces. The side end face intersects with the main end face (which can be perpendicular or non-perpendicular), and the capturing member 122 is mounted on the side end face. As shown in Figure 49, the support member 112 includes a first support wall 1121 and a second support wall 1122 arranged oppositely, and an accommodating chamber S is formed between the first support wall 1121 and the second support wall 1122. The first support wall 1121 has a main end face and a side end face, and the second support wall 1122 has a main end face and a side end face, wherein the side end face M1-1 and the side end face M2-1 are the side end faces of the first support wall 1121, and the side end face M1-2 and the side end face M2-2 are the side end faces of the second support wall 1122. The embodiment of the present disclosure takes two clamping bodies and two support walls as an example. When there are more clamping bodies, more support walls can be arranged on the peripheral side of the distal end of the main body, the inner side faces are relatively arranged to form an accommodating chamber, an opening is formed between adjacent support walls to facilitate the insertion and installation of one end of the clamping member of the clamping body, and the capturing member is mounted on the side end face adjacent to the opening to cooperate with the clamping member to capture the valve leaflets.

Further, the side end face of the support wall of the support member 112 (for example, the side end face M1-1 of the first support wall 1121, the side end face M2-2 of the second support wall 1122) is designed with increased thickness to mount the capturing member 122. For example, the side end face M1-1 of the first support wall 1121 and the side end face M2-2 of the second support wall 1122 of the support member 112 have a first thickness, which is greater than the width of the mounting part 1221 of the capturing member 122. Thus, it is more conducive to the installation of the capturing member 122. As described above, accommodating grooves can be arranged in the side end face M1-1 and the side end face M2-2, so that the mounting part 1221 of the capturing member 122 can be more stably installed in the accommodating grooves.

As described above, as shown in Figure 49, the side end face M1-1 of the first support wall 1121 of the support member 112 has an increased thickness relative to other parts, that is, its thickness is greater than the thickness of other parts of the first support wall 1121 and the thickness of the side end face M2-1 of the second support wall 1122. This thickened design structure can not only better accommodate the capturing member 122, but also the support member 112 has a supporting effect on the installation of the clamping member 121 in the accommodating chamber S. Therefore, the thickened support wall can better support the clamping member 121 and improve the overall stability of the valve clamping device 100. Similarly, the second support wall 1122 can be made into the same structure as the first support wall 1121, that is, with a thickened part, so as to better support the clamping member 121 and further improve the overall stability of the valve clamping device 100. In another embodiment, the thickness of other parts of the first support wall 1121 except the side end face M1-1 can also be made the same as that of the side end face M1-1, so as to further improve the overall stability of the valve clamping device 100. However, considering the balance between the overall size and stability of the valve clamping device 100, the structure shown in Figure 49 has the best balance between stability and size.

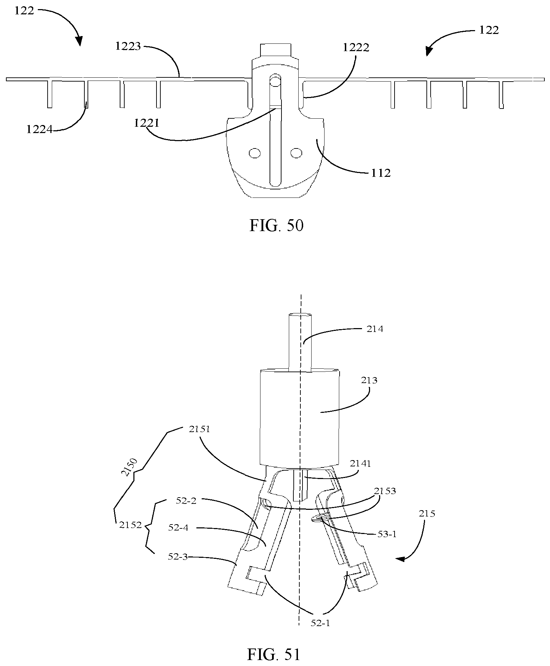

In another embodiment, as shown in Figure 50, the valve clamping device 100 includes an integrally arranged capturing member 122. The mounting parts 1221 of different capturing members 122 are integrally formed into a common mounting part, which is fixedly arranged in the accommodating chamber of the support member 112. The installation method of the capturing member 122 in the support member 112 is not limited, which can be pin joint, clamping, welding, interference fit, etc. For example, similar to the mounting part 1221 in Figure 42 and Figure 44, multiple concave positions can be arranged on the periphery to be clamped at the proximal end of the support member 112, and the clamped positions can be welded. Optionally, multiple grooves can be arranged on the inner wall of the support member 112 at the position where the capturing member 122 is clamped, for the protruding ends at the clamping position of the capturing member 122 to be embedded; that is, the mounting part 1221 of the capturing member 122 includes multiple protruding ends, and the inner wall of the support member 112 has grooves corresponding to the protruding ends, and the protruding ends can be embedded in the grooves, so that the connection of the capturing member is more stable. Optionally, a through hole can be arranged on the mounting part 1221 for the transmission member to pass through the through hole; this installation method is easier to process and has fewer procedures.

Please continue to refer to Figure 32 to Figure 36. In one embodiment, the clamping part 1212 of the clamping member 121 includes a body 1212-1 and clamping leaves 1212-2. The clamping leaves 1212-2 are symmetrically arranged on two sides of the body 1212-1. The clamping member 121 further includes a support part 1213, which is located between the clamping part 1212 and the connecting part 1211. The support part 1213 is located on the side of the body 1212-1 away from the capturing member and extends to the connecting part 1211. Specifically, the connecting part 1211 and the support part 1213 are not arranged at the center of the body 1212-1, but on the side away from the capturing member 122, so that when the side B1 of the connecting part 1211 and the support part 1213 close to the capturing member 122 is in contact with the side B2 of the capturing member 122 (as shown in Figure 48), a compact design can be realized.

Optionally, as shown in Figure 32 to Figure 36, the body 1212-1 of the clamping part 1212 of the clamping member 121 includes at least one hollow 1212-3, the capturing part 1223 of the capturing member 121 is provided with multiple friction elements 1224. When the capturing member 122 is butted with the clamping member 121, the friction elements 1224 extend into the hollow 1212-3. Thus, not only can the weight of the capturing member 121 be reduced, but also the clamping effect can be improved, so that the valve clamping device 100 is easier to be controlled during operation and has a better clamping effect.

Please refer to Figure 46. In some embodiments of the present disclosure, the connection between the bending part 1222 and the mounting part 1221 of the capturing member 122 has a first bend, and the connection between the bending part 1222 and the capturing part 1223 has a second bend, and the width (or cross-sectional size) of the first bend is smaller than that of the second bend. Thus, the bending part 1222 has a curved material reduction, so that when the capturing member 122 cooperates with the clamping member 121 to capture the valve leaflets and perform opening and closing movements, the bending part 1222 has sufficient toughness and low rigidity. In one embodiment, material reduction can be performed at the first bend, with a continuous or discontinuous width increase towards the second bend. For example, there is a width decreasing section from the second bend to the first bend. Similarly, taking two capturing members 122 as an example, for distinction, the first bend and the second bend of the first capturing member are still referred to as the first bend and the second bend, and the first bend and the second bend of the second capturing member are referred to as the third bend and the fourth bend. That is, the connection between the first bending part of the first capturing member and the first mounting part has a first bend, and the connection between the first bending part of the first capturing member and the first capturing part has a second bend, and the width of the first bend is smaller than that of the second bend; and the connection between the second bending part of the second capturing member and the second mounting part has a third bend, and the connection between the second bending part of the second capturing member and the second capturing part has a fourth bend, and the width of the third bend is smaller than that of the fourth bend. In one embodiment, the first bending part includes a width decreasing section from the second bend to the first bend, and the second bending part includes a width decreasing section from the fourth bend to the third bend.

Similar to the above embodiments, in some embodiments of the present disclosure, at least one hollow can be arranged on the bending part to realize another form of material reduction. Thus, when the capturing member 122 cooperates with the clamping member 121 to capture the valve leaflets and perform opening and closing movements, the bending part 1222 has sufficient toughness and low rigidity. These two material reduction methods can be used independently or in combination.

Further, the friction elements 1224 of the capturing member 122 can be arranged in multiple groups. For example, in one embodiment, 1-4 groups are arranged, with 2 friction elements in each group, thus reducing the difficulty of capturing the valve leaflets. In one embodiment, as shown in Figure 42 to Figure 46, the capturing part 1223 of the capturing member 122 includes a first support section 1223-1 and a second support section 1223-2. The first support section 1223-1 is provided with friction elements 1224 facing the clamping member 121, and the second support section 1223-2 is connected between the bending part 1222 and the first support section 1223-1. Several groups of friction elements 1224 are arranged on the first support section 1223-1. The first support section 1223-1 has a multi-segment structure, the size of each segment decreases in the direction away from the second support section 1223-2, and each group of friction elements 1224 is arranged at the position of each segment with the maximum size, and the connection between segments has the minimum size. Thus, the triangular-like design structure of each segment can support the friction element 1224 more stably and improve the clamping performance of the valve clamping device. The last segment is arranged in an arc-shaped structure, which can provide a smooth outer edge, further enabling the valve clamping device 100 to enter the patient's body more smoothly, and effectively reducing the damage of the valve clamping device 100 to the patient or relieving the discomfort of the patient. The present disclosure does not limit the number of friction elements 1224, and four groups are only taken as an example here.

Similarly, taking two capturing members 122 as an example, for distinction, the first support section and the second support section of the first capturing member are still referred to as the first support section and the second support section, and the first support section and the second support section of the second capturing member are referred to as the third support section and the fourth support section. Then, the first capturing part includes a first support section and a second support section, the first support section is provided with a friction element facing the first clamping member, and the second support section is connected between the first bending part and the first support section; and the second capturing part includes a third support section and a fourth support section, the third support section is provided with a friction element facing the second clamping member, and the fourth support section is connected between the second bending part and the third support section. In one embodiment, multiple groups of friction elements are arranged on the first support section, the first support section has a multi-segment structure, the size of each segment decreases in the direction away from the second support section, and each group of friction elements is arranged at the position of each segment with the maximum size, and the connection between segments has the minimum size; multiple groups of friction elements are arranged on the third support section, the third support section has a multi-segment structure, the size of each segment decreases in the direction away from the fourth support section, and each group of friction elements is arranged at the position of each segment with the maximum size, and the connection between segments has the minimum size.

The above embodiments of the present disclosure provide an indirect hinge form, which makes the movement of the clamping member 121 more stable.

For example, as shown in Figure 32 to Figure 36, the clamping member 121 includes a clamping part 1212 and a connecting part 1211. The clamping part 1212 is connected to the connecting part 1211, and the connecting part 1211 is hinged with the second transmission rod 1112. The connecting part 1211 and the clamping part 1212 are connected through a support part 1213. In the embodiment shown in Figure 34, the support part 1213 has a larger cross-sectional area than the connecting part 1211, so that there is a certain connection strength between the connecting part 1211 and the clamping part 1212, so that the clamping part 1212 can clamp the valve leaflets more stably. The arrangement of the support part 1213 can also make the whole clamping member 121 fit the capturing member 122 better. Optionally, an included angle is formed between the connecting part 1211 and the support part 1213, and the included angle is an obtuse angle, which can make the clamping part 1212 fit the capturing member 122 better and have a better capturing effect.

In one embodiment, the clamping member 121 can be integrally formed, thus improving the connection strength between different parts, thereby improving the structural strength of the clamping member 121, and further improving the stability of the valve clamping device 100 during operation.

Similar to the above embodiments, the connecting part 1211 of the clamping member 121 has a moving pin hole 1211-1 and a fixed pin track 1211-2. The support member 112 has a moving pin track 1125. The main body 110 of the valve clamping device 100 further includes a moving pin 115 and a fixed pin 114. The moving pin 115 passes through the moving pin hole 1125 and the distal end of the second transmission rod 1112, and moves along the moving pin track 1125 following the axial motion of the second transmission rod 1112; the fixed pin 114 passes through the fixed pin track 1211-2 and is connected to the support member 112.

Taking a pair of clamping members 121 as an example, the fixed pin 114 passes through the fixed pin track 1211-2 of the connecting part 1211 of a single clamping member 121 to connect the connecting part 1211 with the support member 112. The moving pin 115 passes through the moving pin holes 1211-1 of the connecting parts 1211 of the pair of clamping members 121 to connect the connecting parts 1211 with the support member 112. One end of the moving pin 115 is slidably located in the moving pin track 1125, which is axially located on the first support wall 1121 of the support member 112 and runs through the first support wall 1121; the other end of the moving pin 115 is slidably connected to the second support wall 1122 of the support member 112; or, the moving pin tracks 1125 are axially located on the support walls on both sides of the support member 112 and run through the support walls, so that the moving pin 115 slides axially along the moving pin tracks 1125.

In this embodiment, the opening and closing tracks of the clamping member 121 can be limited by arranging the moving pin track 1125 and the fixed pin track 1211-2, so as to further improve the stability of the valve clamping device 100 during operation.

The operation of the above hinge structure is described below in conjunction with the state of the valve clamping device 100. When the valve clamping device 100 is in the closed state, as shown in Figure 4 and Figure 15, the moving pin 115 is located at the distal end of the moving pin track 1125, and the fixed pin 114 passes through the fixed pin track 1211-2 and is at a certain distance from both ends of the fixed pin track 1211-2. When the second transmission rod 1112 gradually moves towards the proximal end of the valve clamping device 100 along the axial direction, the moving pin 115 will move towards the proximal end of the valve clamping device 100 along the moving pin track 1125, and the clamping member 121 opens and gradually moves away from the main body 110. Figure 5 and Figure 16 show the position where the moving pin 115 moves into the moving pin track 1125 when the clamping member 121 is opened to a certain angle. At this time, if the valve leaflets are captured, the second transmission rod 1112 will gradually move towards the distal end of the valve clamping device 100 along the axial direction, the moving pin 115 follows the second transmission rod 1112 to move towards the distal end of the valve clamping device 100 along the moving pin track 1125, the clamping member 121 is gradually closed, and when the moving pin 115 moves to the distal end of the moving pin track 1125, the valve clamping device 100 returns to the closed state again. If the capture of the valve leaflets fails, the second transmission rod 1112 will gradually move towards the proximal end of the valve clamping device 100 along the axial direction, the moving pin 115 follows the second transmission rod 1112 to move towards the proximal end of the valve clamping device 100 along the moving pin track 1125, and the clamping member 121 is further opened. As shown in Figure 7 and Figure 17, when the moving pin 115 is located at the proximal end of the moving pin track 1125, the valve clamping device 100 is in an inverted state.

Similar to the above embodiments, in some embodiments of the present disclosure, the proximal end of the support member 112 can limit the second transmission rod 1112, and/or the distal end of the support member 112 can limit the second transmission rod 1112. For example, the proximal end of the support member 112 is provided with a channel 1123-1, the size of the channel 1123-1 just allows the second transmission rod 1112 to pass through, so the channel 1123-1 is called a limiting channel, which can limit the radial shaking of the second transmission rod 1112, make the axial motion of the second transmission rod 1112 more stable, and thus make the transmission more stable, which can effectively improve the stability of the valve clamping device 100 during use.

Further, a limiting groove 1124-1 can also be arranged on the inner wall at the distal end of the support member 112. When the distal end of the second transmission rod 1112 moves to the distal end of the support member 112, it can be accommodated in the limiting groove 1124-1. Specifically, when the valve clamping device 100 is in a closed state, that is, when the clamping member 121 is clamped on the main body 110, the distal end of the second transmission rod 1112 is accommodated in the limiting groove 1124-1. The limiting groove 1124-1 can limit the radial shaking of the second transmission rod 1112, which can further improve the stability of the valve clamping device 100 in the closed state and improve the effect of edge-to-edge repair.

In some embodiments of the present disclosure, the main body 110 of the valve clamping device 100 further includes a connecting structure 116 for docking with the delivery system 200. The connection or disconnection between the distal end of the catheter of the delivery system 200 and the proximal end of the main body 110 of the valve clamping device 100 is realized through a clutch mechanism. The clutch mechanism has a clutch device located at the distal end of the catheter and a connecting structure 116 located at the proximal end of the main body 110 of the valve clamping device 100. These two parts cooperate with each other to connect or disconnect under the action of the operating handle at the proximal end of the delivery system 200. The connecting structure 116 can be understood as the part of the clutch mechanism at the proximal end of the main body 110 of the valve clamping device 100, and the above embodiments of the present disclosure do not limit the specific clutch mechanism.

However, in some embodiments, after the implant for transcatheter interventional therapy (for example, the valve clamping device in the above embodiments) is sent to the lesion and implanted, how to release the connection between the implant and the catheter more conveniently and quickly to reduce the operation difficulty for the operator is of great significance.

In view of this, the embodiment of the present disclosure provides a clutch device, which is used to connect the valve clamping device to the distal end of the catheter during the delivery of the valve clamping device, and can realize the quick detachment of the valve clamping device after the implantation is completed. The operation is simple, and it can be applied to complex surgical environments, reducing the injury or discomfort to the patient.

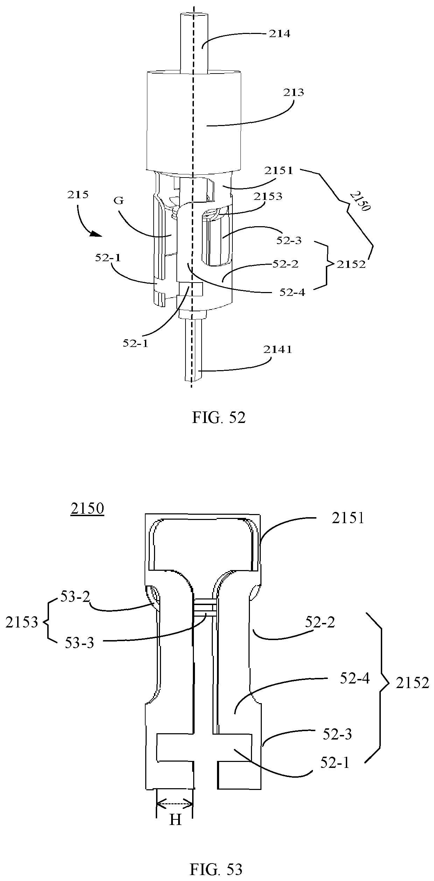

Please refer to Figure 51 and Figure 52, which are schematic perspective views of a clutch device according to an embodiment of the present disclosure, wherein Figure 51 is a schematic diagram of the clutch device in a natural state, and Figure 52 is a schematic diagram of the clutch device under the action of external force. The clutch device 215 is arranged at the distal end of the implantation catheter 213 and is used for connecting or detaching the valve clamping device. The clutch device 215 includes at least two clasping members 2150. The clasping member 2150 includes an elastic part 2151 and a clasping part 2152. The elastic part 2151 is connected between the distal end of the implantation catheter 213 and the clasping part 2152, so that the clasping member 2150 opens relative to the central axis (shown by the dotted line in the figure) of the clutch device 215 in a natural state (i.e., under no external force). Grooves 52-1 are arranged on two sides of the distal end of the clasping part 2152. Under the action of external force, at least two clasping members 2150 are clasped in parallel, a gap G is formed between the side edges of the clasping parts 2152 of adjacent clasping members 2150, and the grooves 52-1 of adjacent clasping members 2150 are butted to form an opening intersecting with the gap G.

Thus, when delivering the valve clamping device, its connecting structure is clamped by the opening formed by the butted grooves, and the valve clamping device is clamped at the distal end of the clutch device. When the external force is removed, the clasping members open in a natural state. Since the opening is formed by butting, the opening quickly releases the clamping of the connecting structure, so that the valve clamping device can be quickly detached. Even in the face of a complex surgical environment, quick detachment can be realized, thereby reducing the injury or discomfort to the patient. The operator can also realize simple and quick operation by simply removing the component applying the external force, and the resistance to be overcome when removing the component applying the external force is smaller, which reduces the difficulty for the operator and improves the surgical effect.

The above clasping members 2150 open at an angle relative to the central axis (shown by the dotted line in the figure) in a natural state, which is, for example, the angle may be an acute angle, ranging from 20° to 40° (including boundary values). Such an arrangement takes into account the detachment efficiency while reducing the impact of the opened clasping members 2150 on the tissues around the implantation position.

Optionally, the opening formed by the butting of the grooves 52-1 of adjacent clasping members 2150 is perpendicular to the gap G between the side edges of the clasping parts 2152 of adjacent clasping members 2150. Thus, when the clasping members are clasped in parallel, a more stable connection can be provided for the valve clamping device, and during detachment, the resistance or interference is less, and the detachment can be realized more quickly.

In some embodiments of the present disclosure, the clasping member 2150 may further include a mating part 2153. The mating part 2153 is arranged at the connection between the elastic part 2151 and the clasping part 2152, and extends towards the interior of the clutch device 215; the mating part 2153 has a through hole 53-1. When the through holes 53-1 of the mating parts 2153 of at least two clasping members 2150 are butted together, the at least two clasping members 2150 are clasped in parallel. When not subjected to external force, the clasping members 2150 open relative to the central axis (shown by the dotted line in the figure). When delivering the valve clamping device 100, the distal end 2141 of the delivery rod 214 passes through the implantation catheter 213 and further extends into the through holes 53-1 of the mating parts 2153, so that different clasping members 2150 are clasped together (as shown in Figure 52). After the implantation of the valve clamping device 100 is completed, the operator can control the delivery rod 214 to be pulled out from the mating parts 2153, and the clasping members 2150 open under no external force to release the connecting structure 116 of the valve clamping device. The mating part 2153 is arranged at the connection between the elastic part 2151 and the clasping part 2152, which can make it easier for the delivery rod 214 to extend into the through hole 53-1.

In other embodiments, the mating part may not be provided, and the external force is provided through a sleeve; when delivering the valve clamping device 100, the sleeve can be sleeved on at least two clasping members 2150, so that the at least two clasping members 2150 are clasped in parallel; after the implantation of the valve clamping device 100 is completed, the operator can control the sleeve to be pulled out from the implantation catheter 213, and the at least two clasping members 2150 open to realize the detachment of the valve clamping device.

Further, please refer to Figure 53 in conjunction, which is a side view of a clutch device according to an embodiment of the present disclosure. The mating part 2153 includes a connecting section 53-2 and a mating section 53-3, and the through hole 53-1 runs through the mating section 53-3. The relative positions of the mating sections 53-3 of the mating parts 2153 of different clasping members 2150 on their respective clasping members 2150 have a position difference of at least the first thickness, so that when at least two clasping members 2150 are clasped in parallel, the mating sections 53-3 can be butted together, so that the through holes 53-1 are butted. The first thickness is the thickness of the mating section 53-3. In one embodiment, the above position difference can be realized by designing different lengths of the connecting sections 53-2 of different mating parts 2153; in another embodiment, the above position difference can be realized by changing the position of the connecting sections of different mating parts on their respective clasping members.

In the embodiment shown above, after the delivery rod 214 is withdrawn from the mating part 2153, the opening is no longer formed at the same time as the clasping member 2150 is opened, becoming independent grooves on different clasping members, and quickly releasing the connecting structure of the valve clamping device.

In some embodiments of the present disclosure, the clasping part 2152 has a hollow structure 52-2, which is arranged between a first position and a second position, wherein the first position is the position of the groove 52-1 on the clasping part 2152, and the second position is the position of the connection between the elastic part 2151 and the clasping part 2152 on the clasping part 2152. Thus, material reduction of the clasping part 2152 can be realized, and when performing opening and closing movements for connecting and detaching the valve clamping device, it has sufficient toughness and low rigidity.

The figure takes two clasping members 2150 as an example, wherein the clasping members 2150 are arranged oppositely; in other embodiments, more clasping members 2150 can be included, and these clasping members are symmetrically distributed on the peripheral side of the catheter 213, or symmetrically arranged relative to the central axis.

In some embodiments of the present disclosure, please continue to refer to Figure 53. The clasping part 2152 has a flat section 52-3, which extends from the hollow structure 52-2 to the distal edge of the clasping part 2152, and both sides of the flat section 52-3 are provided with arc-shaped sections 52-4, and the groove 52-1 is arranged in the arc-shaped section 52-4. The structural design of the flat section can better fit with the connecting part of the valve clamping device when the clutch device is connected to the valve clamping device. Thus, it can not only maintain the stable connection of the valve clamping device in the connected state, but also reduce the resistance during detachment and complete the detachment quickly.

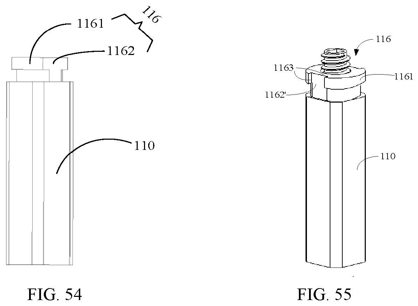

Correspondingly, please refer to Figure 54 and Figure 55, which are schematic diagrams of two connecting structures of the valve clamping device according to the embodiments of the present disclosure. As shown in Figure 54 and Figure 55, the valve clamping device 100 includes a main body 110, the proximal end of the main body 110 is provided with a connecting structure 116, and the connecting structure 116 has a protruding part 1161; as shown in the figure, when delivering the valve clamping device 100, at least two clasping members 2150 of the clutch device 215 are clasped in parallel, the protruding part 1161 of the connecting structure 116 is accommodated in the opening, and the valve clamping device 100 is connected to the clutch device 215; when the implantation of the valve clamping device 100 is completed, at least two clasping members 2150 of the clutch device 215 open in a natural state, the protruding part 1161 of the connecting structure 116 is detached from the opening, and the valve clamping device 100 is detached from the clutch device 215.

As shown in Figure 54, in some embodiments of the present disclosure, the connecting structure 116 has a flat part 1162 to match with the flat section 52-3 of the clasping part 2152 in the above embodiment, so that the clutch device 215 better fits with the connecting structure 116 of the valve clamping device 100. Thus, it can not only maintain the stable connection of the valve clamping device in the connected state, but also reduce the resistance during detachment and complete the detachment quickly.

As shown in Figure 55, in some embodiments of the present disclosure, the two sides of the protruding part 1161 and the intersecting surface 1162' form a clamping groove 1163, and the depth of the clamping groove 1163 matches the thickness of the clasping part 2152 of the clasping member 2150 of the clutch device 215. Further referring to Figure 37, when the clasping members 2150 of the clutch device 215 are clasped in parallel, the edge of the groove 52-1 is clamped with the clamping groove 1163. At this time, the clamping between the clamping groove 1163 of the connecting structure 116 and the edge of the groove 52-1 further improves the connection stability between the clutch device and the valve clamping device.

In the above embodiments, the grooves 52-1 of different clasping members 2150 are symmetrically arranged. In other embodiments, the grooves 52-1 of different clasping members 2150 can be asymmetrically arranged, that is, the sizes of the grooves 52-1 (as shown in Figure 53, that is, the lateral depth H of the groove on the clasping part 2152) are different. Thus, the parts of the connecting structure of the valve clamping device accommodated in the opening in different grooves have the same proportion or different proportions. For example, in two adjacent clasping members, the size of the groove on one clasping member (referred to as the first clasping member for distinction) is larger than the size of the groove on the other clasping member (referred to as the second clasping member for distinction). Thus, when the two clasping members are clasped, the proportion of the connecting structure accommodated in the opening in the first clasping member is larger than that in the second clasping member, and the present disclosure does not limit this proportion.

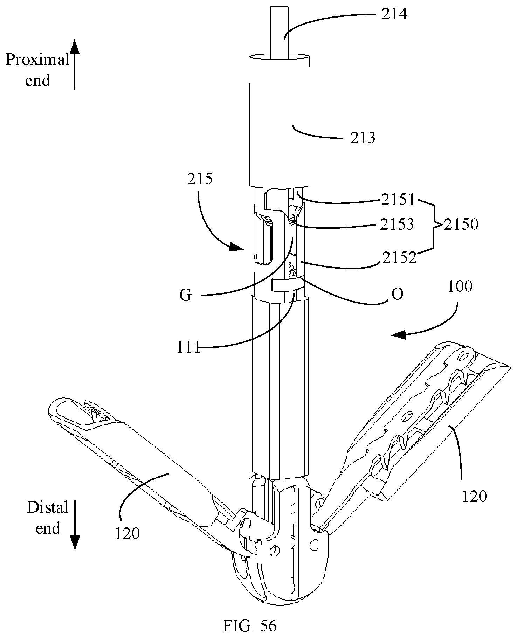

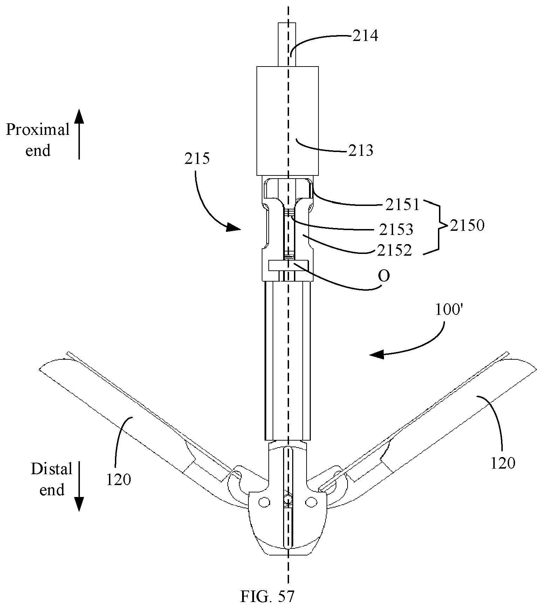

Please refer to Figure 56 and Figure 57, which are respectively a schematic perspective view and a side view of a clutch device connected to a valve clamping device according to an embodiment of the present disclosure. As shown in Figure 56 and Figure 57, the clutch device 215 includes at least two clasping members 2150. The clasping member 2150 includes an elastic part 2151 and a clasping part 2152. The elastic part 2151 is connected between the distal end of the implantation catheter 213 and the clasping part 2152, so that the clasping member 2150 opens relative to the central axis in a natural state (i.e., under no external force). The distal end of the clasping part 2152 is provided with an opening O (when the clasping members 2150 are clasped with each other). The opening O matches the connecting structure 116 of the valve clamping device. When the clasping members 2150 are clasped on the proximal end of the valve clamping device under the action of external force, different clasping members 2150 are clasped in parallel, and a gap G is formed between the edges of the clasping parts 2152 of adjacent clasping members 2150. The protruding part 1161 of the connecting structure 116 of the valve clamping device extends into the opening O, clamping the valve clamping device at the distal end of the clutch device 215.

Compared with the existing clutch device, the clutch device in the present disclosure connects the valve clamping device by clasping, the clutch device 215 is easier to detach after the valve clamping device is implanted.

The formation of the opening O can be realized by arranging the groove 52-1 at the distal end of the clasping member 2150 in the above embodiment, so that the opening O can be formed by butting when the clasping members 2150 are clasped in parallel. In other embodiments, the opening O can also be directly formed at a non-edge position of the clasping part 2152 when the clasping members 2150 are not clasped.

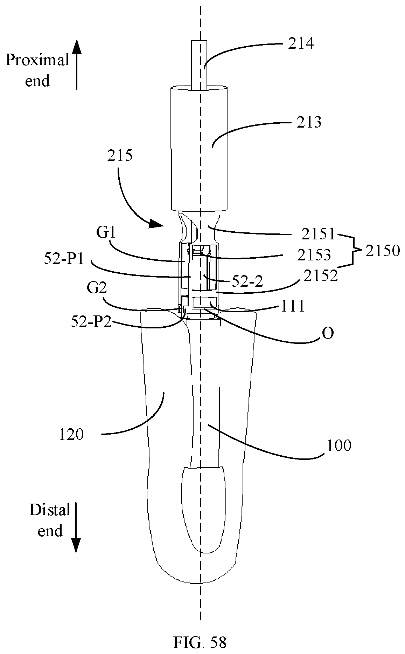

For example, please refer to Figure 58, which is a schematic perspective view of another clutch device connected to a valve clamping device according to an embodiment of the present disclosure. The difference between the clutch device and the previous embodiment is that the opening O is completely and directly arranged at the distal end of the clasping part 2152. As shown in Figure 58, the clutch device 215 includes an elastic part 2151 and a clasping part 2152. The elastic part 2151 is connected between the distal end of the catheter 213 and the clasping part 2152, so that the clasping member 2150 opens relative to the central axis of the clutch device in a natural state, and under the action of external force, at least two clasping members 2150 are clasped in parallel. The clasping part 2152 includes a first segment 52-P1 and a second segment 52-P2. The proximal end of the first segment 52-P1 is connected to the elastic part 2151, the distal end of the first segment 52-P1 is connected to the second segment 52-P2, and the width of the second segment 52-P2 is larger than that of the first segment 52-P1. Thus, when at least two clasping members 2150 are clasped in parallel, the width of the first gap G1 formed between the side edges of the first segments 52-P1 of the clasping parts 2152 of adjacent clasping members 2150 is larger than the width of the second gap G2 formed between the side edges of the second segments 52-P2 of the clasping parts 2152 of adjacent clasping members 2150, and the proportion of the second segment 52-P2 in the clasping part 2152 in the first direction is less than or equal to 1/3, for example, 10%-30% (including boundary values), wherein the first direction is the direction in which the clasping part 2152 extends from the proximal end connected to the elastic part 2151 to the distal end of the clasping part 2152.

When the above clutch device is connected to the valve clamping device, the second segments 52-P2 provide a larger area of clasping contact to improve the connection stability of the valve clamping device, and a larger gap G1 is formed between the first segments 52-P1, which reduces interference during detachment and completes the detachment of the valve clamping device more quickly. Thus, both stability and the convenience of detachment operation can be taken into account.

Further, the ratio of the width of the second segment 52-P2 to the width of the first segment 52-P1 is 110%-150% (including boundary values), that is, the width of the second segment is 10%-50% more than that of the first segment, so as to further improve the connection stability of the valve clamping device during delivery. The width of the second gap G2 can be zero, that is, when adjacent clasping members 2150 are clasped in parallel, the side edges of the second segments 52-P2 are in contact, so as to increase the area of clasping contact and further improve the stability. Or, as shown in Figure 58, the width of the second gap G2 can be greater than zero, so that the detachment of the valve clamping device can be completed more quickly.

The position of the above opening O can have various implementations: in one embodiment, the opening O is located at the junction of the first segment 52-P1 and the second segment 52-P2, and the proximal edge of the opening O is located in the first segment 52-P1, and the distal edge is located in the second segment 52-P2; in another embodiment, the opening O is located in the first segment 52-P1, and the distal edge of the opening O is located at or near the junction of the first segment 52-P1 and the second segment 52-P2; in yet another embodiment, the opening O is located in the second segment 52-P2, and the proximal edge of the opening O is located at or near the junction of the first segment 52-P1 and the second segment 52-P2.

In other embodiments, the first gap can also be used to clamp the connecting structure of the valve clamping device, so that there is no need to set an opening. For example, the width of the first gap G1 matches the protruding part 1161 of the connecting structure 116 of the valve clamping device. When the clutch device 215 is connected to the valve clamping device 100, the protruding part 1161 is accommodated in the first gap G1 and abuts against the second segment 52-P2. Thus, similar to the embodiment shown in Figure 56 and Figure 57 above, the first gap G1 is formed by the butting of the adjacent clasping members 2150. After the external force is removed, the first gap G1 and the second segment 52-P2 quickly release the clamping of the connecting structure 116, so that the valve clamping device 100 can be quickly detached. Even in the face of a complex surgical environment, quick detachment can be realized, thereby reducing the injury or discomfort to the patient. The operator can also realize simple and quick operation by simply removing the component applying the external force, and the resistance to be overcome when removing the component applying the external force is smaller, which reduces the difficulty for the operator and improves the surgical effect.

Further, the clutch device 215 can also have a structure similar to that of the clutch device in the above embodiments, such as a mating part 2153 and a hollow structure 52-2. The difference is that the hollow structure 52-2 is located in the first segment 52-P1. In one embodiment, the ratio of the width of the hollow structure 52-2 to the width of the first segment 52-P1 is 40%-90% (including boundary values). Thus, the connection stability of the clasping part 2152 to the valve clamping device can be met, and the width of the first gap G1 can be maximized, thereby improving the convenience of detachment.

Correspondingly, as shown in Figure 2, the embodiment of the present disclosure also provides a delivery system 200 and a valve repair system including the delivery system. The delivery system 200 includes an implantation catheter 213, and any of the above clutch devices 215, which is arranged at the distal end of the implantation catheter for connecting or detaching the valve clamping device 100. Further, the delivery system 200 also includes a delivery rod 214, which is configured to pass through the implantation catheter 213 and is used for providing the external force acting on the clutch device 215; when delivering the valve clamping device 100, the distal end of the delivery rod 214 applies external force to the clasping members 2150 of the clutch device 215, so that at least two clasping members 2150 are clasped in parallel; when detaching the valve clamping device 100, the delivery rod 214 withdraws the external force from the clasping members 2150 of the clutch device 215. For example, when the delivery rod 214 passes through the through holes 53-1 of the mating parts 2153 of at least two clasping members 2150, the through holes 53-1 of the mating parts 2153 are butted together, and at least two clasping members 2150 are clasped in parallel.

In the above-mentioned embodiments, the description of each embodiment has its own emphasis. For parts that are not described or recorded in detail in a certain embodiment, reference may be made to the relevant descriptions of other embodiments.

It should be noted that the above embodiments can be freely combined as required. The above are only part of the implementations of the present disclosure. For those of ordinary skill in the art, without departing from the principle of the present disclosure, several improvements and modifications can be made, and these improvements and modifications should still fall within the protection scope of the present disclosure.

## Claims

1. A valve clamping device, comprising:
a main body;
a clamping body, mounted on the main body;
the main body comprises a transmission assembly, the transmission assembly comprises a first transmission rod and a second transmission rod, a transmission structure is arranged at a distal end of the first transmission rod and a proximal end of the second transmission rod, and the transmission structure transmits an axial motion between the first transmission rod and the second transmission rod;
the clamping body comprises a clamping member, the clamping member is hinged to a distal end of the second transmission rod, and the axial motion of the second transmission rod drives the opening and closing motions of the clamping member, so as to change an included angle between the clamping member and the main body.

2. The valve clamping device according to claim 1, **characterized in that**,
a driving force for controlling the valve clamping device acts radially on the first transmission rod, and the transmission structure transmits the axial motion while preventing the transmission of a radial motion tendency to the second transmission rod.

3. The valve clamping device according to claim 1 or 2, **characterized in that**,
the transmission structure comprises a first limiting structure and a second limiting structure;
the first limiting structure is arranged at the distal end of the first transmission rod, the second limiting structure is arranged at the proximal end of the second transmission rod, and the first limiting structure and the second limiting structure axially abut against each other when the first transmission rod or the second transmission rod moves axially.

4. The valve clamping device according to claim 3, **characterized in that**,
the axial abutment between the first limiting structure and the second limiting structure restricts the relative movement between the first transmission rod and the second transmission rod in one axial direction, or the axial abutment between the first limiting structure and the second limiting structure restricts the relative movement between the first transmission rod and the second transmission rod in two axial directions.

5. The valve clamping device according to claim 4, **characterized in that**,
the second limiting structure comprises a concave side surface, the first limiting structure comprises an extension part extending towards the interior of the first transmission rod, and the extension part is configured to be located in a groove of the concave side surface.

6. The valve clamping device according to claim 4, **characterized in that**,
the first limiting structure comprises a first limiting member arranged at the distal end of the first transmission rod, the first limiting member comprises a first chamber, the first chamber has a first opening towards an distal end of the valve clamping device, the proximal end of the second transmission rod is configured to pass through the first opening towards the distal end of the valve clamping device, the second limiting structure at the proximal end of the second transmission rod is configured to be accommodated in the first chamber, and a maximum size of the second limiting structure is larger than the size of the first opening towards the distal end of the valve clamping device; or,
the second limiting structure comprises a second limiting member arranged at the proximal end of the second transmission rod, the second limiting member comprises a second chamber, the second chamber comprises a second opening towards a proximal end of the valve clamping device, the distal end of the first transmission rod is configured to pass through the second opening towards the proximal end of the valve clamping device, the first limiting structure at the distal end of the first transmission rod is configured to be accommodated in the second chamber, and a maximum size of the first limiting structure is larger than the size of the opening towards the proximal end of the valve clamping device.

7. The valve clamping device according to claim 6, **characterized in that**,
the second limiting structure which is configured to be located in the first chamber of the first limiting member is spherical or ellipsoidal; or,
the first limiting structure which is configured to be located in the second chamber of the second limiting member is spherical or ellipsoidal.

8. The valve clamping device according to claim 6, **characterized in that**,
the first limiting structure comprises a third limiting member arranged at the distal end of the first transmission rod, a third chamber of the third limiting member further comprises an opening towards the proximal end of the valve clamping device, and the distal end of the first transmission rod is configured to pass through the opening towards the proximal end of the valve clamping device and abut against the second limiting structure at the proximal end of the second transmission rod in the third chamber during an axial motion towards the distal end of the valve clamping device; or,
the second limiting structure comprises a fourth limiting member arranged at the proximal end of the second transmission rod, a fourth chamber of the fourth limiting member further comprises an opening towards the distal end of the valve clamping device, and the proximal end of the second transmission rod is configured to pass through the opening towards the distal end of the valve clamping device and abut against the first limiting structure at the proximal end of the first transmission rod in the fourth chamber during an axial motion towards the distal end of the valve clamping device.

9. The valve clamping device according to claim 3, **characterized in that**,
the first limiting structure is integrally formed with the first transmission rod, and the second limiting structure is integrally formed with the second transmission rod; or,
the first limiting structure is integrally formed with the first transmission rod, and the second limiting structure is fixedly connected to the second transmission rod; or,
the first limiting structure is fixedly connected to the first transmission rod, and the second limiting structure is integrally formed with the second transmission rod; or,
the first limiting structure is fixedly connected to the first transmission rod, and the second limiting structure is fixedly connected to the second transmission rod.

10. The valve clamping device according to claim 1, **characterized in that**,
at least one of surfaces in the transmission assembly abutting against each other is provided with a convex structure.

11. The valve clamping device according to claim 1, **characterized in that**,
the first transmission rod comprises an inner chamber, and a cross-section of the inner chamber is non-circular.

12. The valve clamping device according to claim 11, **characterized in that**,
the cross-section of the inner chamber is elliptical, rectangular, triangular, or racetrack-shaped.

13. The valve clamping device according to claim 1, **characterized in that**,
the main body further comprises:
an attachment member, the first transmission rod is arranged in the attachment member, an inner side wall of the attachment member is provided with an internal thread, an outer side wall of the first transmission rod is provided with an external thread, and the internal thread matches the helix of the external thread.

14. The valve clamping device according to claim 1, **characterized in that**,
the clamping body further comprises:
a capturing member arranged between the main body and the clamping member.

15. A valve repair system, comprising:
the valve clamping device according to any one of claims 1 to 14;
a delivery system, for delivering and controlling the valve clamping device.

16. The valve repair system according to claim 15, **characterized in that**,
the delivery system comprises a catheter and a delivery device, the delivery device comprises a control mechanism and a delivery rod, a proximal end of the delivery rod is connected to the control mechanism, a distal end of the delivery rod passes through the catheter and acts radially on the transmission assembly of the valve clamping device, and a cross-section of the distal end of the delivery rod is non-circular.

17. The valve repair system according to claim 16, **characterized in that**,
a surface of the delivery rod abutting against the second transmission rod of the transmission assembly of the valve clamping device is provided with a convex structure.
